(19) Europäisches Patentamt / European Patent Office / Office européen des brevets

(11) **EP 2 348 986 B1**

(12) **EUROPEAN PATENT SPECIFICATION**

(45) Date of publication and mention
of the grant of the patent:
**08.07.2015 Bulletin 2015/28**

(21) Application number: **09824286.0**

(22) Date of filing: **10.11.2009**

(51) Int Cl.:
*A61B 5/053* (2006.01)

(86) International application number:
**PCT/AU2009/001461**

(87) International publication number:
**WO 2010/051600 (14.05.2010 Gazette 2010/19)**

(54) **FLUID INDICATOR**

FLUIDINDIKATOR

INDICATEUR DE FLUIDE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **10.11.2008  AU 2008905783**

(43) Date of publication of application:
**03.08.2011  Bulletin 2011/31**

(73) Proprietor: **Impedimed Limited
Pinkenba, QLD 4008 (AU)**

(72) Inventors:
• **ESSEX, Tim
Clayfield
Queensland 4011 (AU)**
• **WARD, Leigh, Cordwin
Kenmore Hills
Queensland 4069 (AU)**
• **GAW, Richelle, Leanne
Greenslopes
Queensland 4210 (AU)**

(74) Representative: **Gray, Peter John Bracey
Thompson Gray LLP
Central Court
25 Southampton Buildings
London WC2A 1AL (GB)**

(56) References cited:
EP-A1- 1 080 686          WO-A1-00/79255
WO-A1-2005/122888         WO-A1-2007/002992
WO-A1-2007/014417         WO-A1-2008/064426
WO-A1-2009/100491         US-A1- 2001 020 138
US-B2- 6 643 543

• NAWARYCZ T ET AL: "TRIPLE-FREQUENCY ELECTROIMPEDANCE METHOD FOR EVALUATION OF BODY WATER COMPARTMENTS", MEDICAL & BIOLOGICAL ENGINEERING & COMPUTING, STEVENHAGE, HERTS, GB, vol. 34, no. SUPP. 01. PT 02, 1 January 1996 (1996-01-01), page 181/182, XP000965197,
• THOMAS B J ET AL: "Bioimpedance Spectrometry in the Determination of Body Water Compartments: Accuracy and Clinical Significance", APPLIED RADIATION AND ISOTOPES, ELSEVIER, OXFORD, GB, vol. 49, no. 5-6, 6 May 1998 (1998-05-06), pages 447-455, XP004110545, ISSN: 0969-8043, DOI: 10.1016/S0969-8043(97)00052-3

EP 2 348 986 B1

## Description

### Background of the Invention

The present invention relates to a method and apparatus for use in analysing impedance measurements performed, and in particular to a method and apparatus for determining fluid levels within a segment of a subject.

### Description of the Prior Art

The reference in this specification to any prior publication (or information derived from it), or to any matter which is known, is not, and should not be taken as an acknowledgment or admission or any form of suggestion that the prior publication (or information derived from it) or known matter forms part of the common general knowledge in the field of endeavour to which this specification relates.

One existing technique for determining biological indicators relating to a subject, such as cardiac function, body composition, and other health status indicators, such as the presence of oedema, involves the use of bioelectrical impedance. This process typically involves using a measuring device to measure the electrical impedance of a subject's body using a series of electrodes placed on the skin surface. Changes in electrical impedance measured at the body's surface are used to determine parameters, such as changes in fluid levels, associated with the cardiac cycle, oedema, or the like.

WO0079255 describes a method and apparatus for assessing tissue for the presence of oedema and, in particular, lymphoedema. The method is based on measurement of bioelectrical impedance at a single low frequency voltage. Measurements are taken of two anatomical regions of a subject and analysed to give an indication of tissue oedema.

WO2005122888 describes a method of detecting tissue oedema in a subject. The method includes determining a measured impedance for first and second body segments. An index indicative of a ratio of the extra-cellular to intra-cellular fluid is then calculated for each body segment, with these being used to determine an index ratio based on the index for the first and second body segments. The index ratio can in turn be used to determine the presence, absence or degree of tissue oedema, for example by comparing the index ratio to a reference or previously determined index ratios.

EP1080686A1 describes judging body composition based on the measured results of a bioelectrical impedance measuring method where a first, second and third bioelectrical impedance values are determined by a measurement using alternating currents having a first, second and third frequencies respectively, and where then a vector impedance locus is derived from only the derived first, second and third bioelectrical impedance values to determine the bioelectrical impedance values at zero frequency and at an infinite frequency.

### Summary of the Present Invention

The present invention seeks to substantially overcome, or at least ameliorate, one or more disadvantages of existing arrangements.

In a first broad form the present invention provides apparatus for use in performing impedance measurements on a subject, wherein the apparatus includes a processing system for:

a) determining impedance measurements of at least one body segment of the subject at more than three frequencies;
b) determining combinations of the impedance measurements, each combination including impedance measurements at three frequencies;
c) for each combination:

i. at each of the three frequencies, determining first and second parameter values for first and second impedance parameters relating to the impedance measurements;
ii. solving simultaneous equations representing a circle defined with respect to the first and second impedance parameters to thereby determine circle parameter values, the equations being solved using the first and second parameter values at each of the three frequencies; and
iii. using the circle parameter values to determine a third impedance parameter value at a respective frequency; and,

d) determining an indicator indicative of relative fluid levels within the body segment of the subject, the indicator being an average of the third impedance parameter values determined for each combination.

Typically the indicator is indicative of extra-cellular fluid levels.

Typically the third impedance parameter is indicative of the impedance at zero frequency.

Typically the processing system is for:

a) determining third impedance parameter values at a respective frequency for each of first and second body segments;

b) determining a ratio using the third impedance parameter values; and,

c) using the ratio to determine the indicator.

Typically the first and second body segments are portions of contra-lateral limbs.

Typically the processing system is for:

a) comparing the ratio to a reference; and,

b) using results of the comparison to determine the indicator.

Typically the reference includes at least one of:

a) a predetermined threshold;

b) a tolerance determined from a normal population;

c) a predetermined range; and,

d) an indicator previously determined for the subject.

Typically the processing system is for:

a) using the circle parameter values to determine a fourth impedance parameter value at a respective frequency;

b) using the third and fourth impedance parameters values to determine an index indicative of a ratio of the extra-cellular to intra-cellular fluid; and,

c) determining the indicator using the index.

Typically the processing system is for:

a) determining an index for first and second body segments; and,
b) determining an index ratio based on the index for the first and second body segments.

Typically the first and second body segments are at least one of: different types of body segment, limbs and a leg and an arm.

Typically the processing system is for:

a) determining values for parameters $R_0$ and $R_\infty$ from the impedance parameter values; and,
b) calculating an index (I) using the equation:

$$I = \frac{R_\infty}{R_0 - R_\infty}$$

where:

$R_0$ is the resistance at zero frequency; and,
$R_\infty$ is the resistance at infinite frequency.

Typically the processing system is for, displaying an indication of at least one of:

a) the third impedance parameter values;
b) the first and second impedance parameter values;
c) the circle parameters;
d) a ratio of extra-cellular to intra-cellular fluid; and,
e) an indication of the at least one of the presence, absence or degree of tissue oedema in the subject.

Typically the apparatus includes:

a) a signal generator for generating an alternating signal at each of a plurality of frequencies;
b) at least two supply electrodes for applying the generated alternating signal to a subject;
c) at least two measurement electrodes for detecting a signal across the subject; and,
d) a sensor coupled to the measurement electrodes for determining the signal across the subject, the sensor being coupled to the processing system to thereby allow the processing system to determine the measured impedances.

Typically the apparatus includes a number of electrode systems, and wherein each electrode system includes:

a) a sensor;

b) a signal generator;

c) a first substrate having the signal generator and sensor mounted thereon; and,

d) a second substrate having at least two conductive pads mounted thereon, the conductive pads forming a first and a second electrode for coupling the signal generator and the sensor to a subject in use.

Typically the electrode system includes a capacitive cancelling circuit for cancelling capacitive coupling between the first and second electrodes;
preferably the capacitive cancelling circuit includes an inverting amplifier for coupling a signal generator output to a sensor input and the inverting amplifier applies a capacitive cancelling signal to the sensor input to thereby cancel any effective capacitance between the first electrode and the second electrode.
Typically an inverting amplifier output is coupled to the sensor input via at least one of:

a) a resistor;

b) a capacitor; and,

c) an inductor.

Typically at least one of the resistor and capacitor are adjustable, thereby allowing a capacitive cancelling signal applied to the sensor input to be controlled.
Typically the electrode system includes an input capacitance cancelling circuit for cancelling an effective input capacitance at a sensor input.
Typically the electrode system includes a feedback loop for connecting a sensor output to the sensor input.
Typically the feedback loop includes at least one of:

a) a resistor;

b) a capacitor; and,

c) an inductor.

Typically at least one of the resistor and capacitor are adjustable, thereby allowing a current flow from the sensor output to the sensor input to be controlled.
Typically the feedback loop applies an input capacitance cancelling signal to the sensor input to thereby cancel any effective capacitance at the sensor input.
Typically the processing system includes a memory for storing software, and a processor operating under control of the software stored in the memory, and wherein the processor:

a) determines impedance measurements of at least one body segment of the subject at more than three frequencies;
b) determines combinations of the impedance measurements, each combination including impedance measurements at three frequencies;
c) for each combination:

i. determines, at each of the three frequencies, first and second parameter values for first and second impedance parameters relating to the impedance of measurements;
ii. solves simultaneous equations representing a circle defined with respect to the first and second impedance parameters to thereby determine circle parameter values, the equations being solved using the first and second parameter values at each of the three frequencies;
iii. uses the circle parameter values to determine a third impedance parameter value at a respective frequency; and,

d) determines an indicator indicative of relative fluid levels within the body segment of the subject, the indicator being an average of the third impedance parameter values determined for each combination.

In a second broad form the present invention provides a method for use in performing impedance measurements on a subject, wherein the method includes, in a processing system:

a) determining impedance measurements of at least one body segment of the subject at more than three frequencies;
b) determining combinations of the impedance measurements, each combination including impedance measurements at three frequencies;
c) for each combination:

i. at each of the three frequencies, determining first and second parameter values for first and second impedance parameters relating to the impedance measurements;
ii. solving simultaneous equations representing a circle defined with respect to the first and second impedance parameters to thereby determine circle parameter values, the equations being solved using the first and second parameter values at each of the three frequencies; and
iii. using the circle parameter values to determine a third impedance parameter value at a respective frequency; and,

d) determining an indicator indicative of relative fluid levels within the body segment of the subject, the indicator being an average of the third impedance parameter values determined for each combination.

It will be appreciated that the broad forms of the invention may be used individually or in combination, and may be used for diagnosis of the presence, absence or degree of a range of conditions and illnesses, including, but not limited to oedema, lymphoedema, body composition and the like.

**Brief Description of the Drawings**

An example of the present invention will now be described with reference to the accompanying drawings, in which: -

Figure 1 is a schematic diagram of an example of an impedance measuring device;
Figure 2 is a flowchart of an example of a process for determining fluid levels in a segment of a subject;
Figure 3 is a schematic of an example of a theoretical equivalent circuit for biological tissue;
Figure 4 is an example of a locus of impedance known as a Cole-Cole plot;
Figure 5 is a flowchart of a second example of a process for determining fluid levels in a subject;
Figures 6A and 6B are diagrams of examples of electrode positions for use in measuring limb impedances;
Figures 6C and 6D are schematic diagrams of examples of electrode positions for use in measuring limb impedances;
Figure 7 is a schematic of an example of the functionality of the processing system of Figure 1; Figures 8A to 8C are a flowchart of an example of a process for performing impedance measurements using the apparatus of Figure 7;
Figure 9A is a schematic diagram of an example of an electrode system incorporating a signal generator and a sensor;
Figure 9B is a schematic diagram illustrating cross electrode capacitive coupling;
Figure 9C is a schematic diagram of an example of a cross electrode capacitance cancelling circuit;
Figure 9D is a schematic diagram of an example of an input capacitance cancelling circuit; Figure 10 is a schematic diagram of an example of lead connections between the measuring device and the electrode system of Figure 9A;

Figure 11 is a schematic diagram of an example of a lead arrangement;
Figures 12A and 12B are schematic diagrams of examples of electrode configurations used during balancing; and,
Figure 12C is a schematic diagram of effective electrical models for the electrode arrangements of Figures 12A and 12B.

**Detailed Description of the Preferred Embodiments**

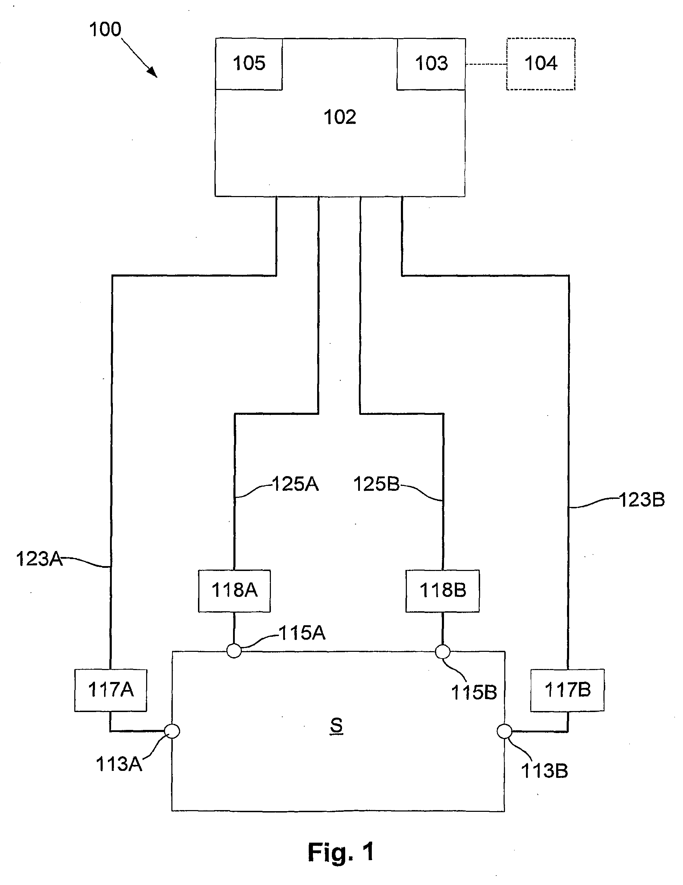

An example of apparatus suitable for performing an analysis of a subject's bioelectric impedance will now be described with reference to Figure 1.

As shown the apparatus includes a measuring device 100 including a processing system 102, connected to one or more signal generators 117A, 117B, via respective first leads 123A, 123B, and to one or more sensors 118A, 118B, via respective second leads 125A, 125B. The connection may be via a switching device, such as a multiplexer, although this is not essential.

In use, the signal generators 117A, 117B are coupled to two first electrodes 113A, 113B, which therefore act as drive electrodes to allow signals to be applied to the subject S, whilst the one or more sensors 118A, 118B are coupled to the second electrodes 115A, 115B, which act as sense electrodes, allowing signals across the subject S to be sensed.

The signal generators 117A, 117B and the sensors 118A, 118B may be provided at any position between the processing system 102 and the electrodes 113A, 113B, 115A, 115B, and may be integrated into the measuring device 100. However, in one example, the signal generators 117A, 117B and the sensors 118A, 118B are integrated into an electrode system, or another unit provided near the subject S, with the leads 123A, 123B, 125A, 125B connecting the signal generators 117A, 117B and the sensors 118A, 118B to the processing system 102.

It will be appreciated that the above described system is a two channel device, used to perform a classical four-terminal impedance measurement, with each channel being designated by the suffixes A, B respectively. The use of a two channel device is for the purpose of example only, as will be described in more detail below.

An optional external interface 103 can be used to couple the measuring device 100, via wired, wireless or network connections, to one or more peripheral devices 104, such as an external database or computer system, barcode scanner, or the like. The processing system 102 will also typically include an I/O device 105, which may be of any suitable form such as a touch screen, a keypad and display, or the like.

It will be appreciated that in practice, the processing system 102 will typically include a memory, or other store, for storing software. The software provides instructions that cause a processor within the processing system to perform the processes required to perform and/or interpret impedance measurements, as will be described in more detail below.

In use, the processing system 102 is adapted to generate control signals, which cause the signal generators 117A, 117B to generate one or more alternating signals, such as voltage or current signals of an appropriate waveform, which can be applied to a subject S, via the first electrodes 113A, 113B. The sensors 118A, 118B then determine the voltage across or current through the subject S, using the second electrodes 115A, 115B and transfer appropriate signals to the processing system 102.

Accordingly, it will be appreciated that the processing system 102 may be any form of processing system which is suitable for generating appropriate control signals and at least partially interpreting the measured signals to thereby determine the subject's bioelectrical impedance, and optionally determine other information such as the presence, absence or degree of conditions, such as oedema, lymphoedema, measures of body composition, cardiac function, or the like.

The processing system 102 may therefore be a suitably programmed computer system, such as a laptop, desktop, PDA, smart phone or the like. Alternatively the processing system 102 may be formed from specialised hardware, such as an FPGA (field programmable gate array), or a combination of a programmed computer system and specialised hardware, or the like.

In use, the first electrodes 113A, 113B are positioned on the subject to allow one or more signals to be injected into the subject S. The location of the first electrodes will depend on the segment of the subject S under study. Thus, for example, the first electrodes 113A, 113B can be placed on the thoracic and neck region of the subject S to allow the impedance of the chest cavity to be determined for use in cardiac function analysis. Alternatively, positioning electrodes on the wrist and ankles of a subject allows the impedance and hence fluid levels in the limbs and/or the entire body to be determined, for use in oedema analysis, or the like.

Once the electrodes are positioned, one or more alternating signals are applied to the subject S, via the first leads 123A, 123B and the first electrodes 113A, 113B. The nature of the alternating signal will vary depending on the nature of the measuring device and the subsequent analysis being performed.

Typically the system uses Multiple Frequency Bioimpedance Analysis (MFBIA) in which multiple signals, each having a respective frequency are injected into the subject S, with the measured impedances being used in the assessment of fluid levels.

In one example, the applied signal is generated by a voltage generator, which applies an alternating voltage to the subject S, although alternatively current signals may be applied. In one example, the voltage source is typically symmetrically arranged, with each of the signal generators 117A, 117B being independently controllable, to allow the signal voltage across the subject to be varied.

A voltage difference and/or current is measured between the second electrodes 115A, 115B. In one example, the voltage is measured differentially, meaning that each sensor 118A, 118B is used to measure the voltage at each second electrode 115A, 115B and therefore need only measure half of the voltage as compared to a single ended system.

The acquired signal and the measured signal will be a superposition of voltages generated by the human body, such as the ECG (electrocardiogram), voltages generated by the applied signal, and other signals caused by environmental electromagnetic interference. Accordingly, filtering or other suitable analysis may be employed to remove unwanted components.

The acquired signals are then used to determine first and second parameter values, such as resistance and reactance values, at each frequency. In one example, this is achieved using an algorithm to derive an amplitude and phase signal at each frequency, with these values in turn being used to derive the resistance and reactance values.

As part of the above described process, the distance between the second electrodes 115A, 115B may be measured and recorded. Similarly, other parameters relating to the subject may be recorded, such as the height, weight, age, sex, health status, any interventions and the date and time on which they occurred. Other information, such as current medication, may also be recorded. This can then be used in performing further analysis of the impedance measurements, so as to allow determination of the presence, absence or degree of oedema, to assess body composition, or the like.

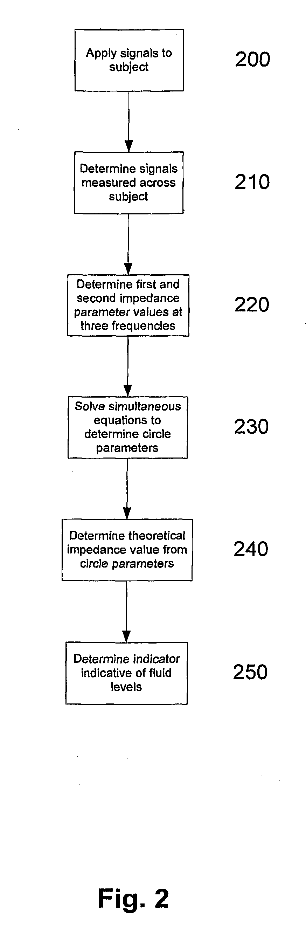

An example of a processor for determining a fluid level indicator will now be described with reference to Figure 2.

In this example, the first stage is for impedance measurements to be performed on at least a body segment of the subject. This is typically achieved by having the processor control the signal generators 117A, 117B, to cause the signal generators to apply an electrical signal having a known frequency to the subject S at step 200 with electrical signals across and/or through the subject being measured at step 210 using the sensors 118A, 118B. It will be appreciated that whilst an indication of both the current flow through the subject and voltage across the subject are required to calculate an impedance, it is not necessary to measure both of these as one may be derived based on information regarding the signals applied to the subject.

The impedance measurements are performed at at least three frequencies. Indications of the signals are used by the processor to determine first and second impedance parameter values at each of the frequencies, at step 220. The nature of the impedance parameter values will vary depending on the preferred implementation. Thus, for example the impedance parameter values could include magnitude and phase information relating to the measured signals. However, in one example the impedance parameter values are indicative of the resistance and reactance, as derived from the magnitude and phase signals.

At step 230 simultaneous equations are solved by the processor using the first and second impedance parameter values determined at each of the three frequencies, thereby allowing circle parameters to be determined. The circle parameters are used to define a locus corresponding to at least part of an arc of a circle in a space defined by the parameter values. Thus, in one example, the simultaneous equations represent a circular locus provided in a reactance/resistance space, also commonly referred to as a Cole plot or Wessel plot as will be described in more detail below.

At step 240 the processor determines a theoretical impedance value from the circle parameters. Whilst any theoretical impedance value may be determined, in one example, this is an impedance value representing a low frequency impedance. The impedance value is typically indicative of the impedance that will be obtained for an applied signal of less than 500 kHz, typically less than 50 kHz and preferably at 0 kHz, often referred to as $R_0$.

At step 250, the theoretical impedance value is used by the processor in determining an indicator indicative of fluid levels. In one example the impedance at low frequency is directly proportional to the amount of extracellular fluid (ECF) in the subject and accordingly, the theoretical impedance value can simply be used directly as the indicator of extracellular fluid levels. However, this may be combined with information, such as impedance values from other body segments, allowing a relative extracellular fluid level within the body segment, as compared to a reference body segment, to be established.

Additionally, other information, such as the resistance at infinite frequency may be used allowing an index representing the ratio of intra to extracellular fluids to be determined, as will be described in more detail below.

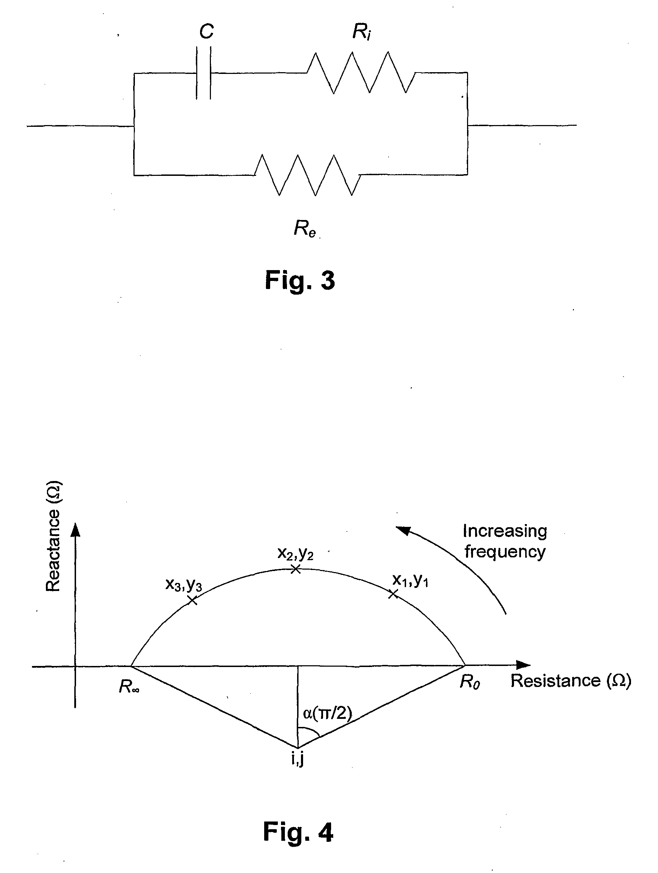

Figure 3 is an example of an equivalent circuit that effectively models the electrical behaviour of biological tissue. The equivalent circuit has two branches that represent current flow through extracellular fluid and intracellular fluid (ICF). The extracellular component of biological impedance is represented by $R_e$ and the intracellular component is represented by $R_i$. Capacitance of the cell membrane in the intracellular path is represented by C.

The relative magnitudes of the extracellular and intracellular components of impedance of an alternating current (AC) are frequency dependent. At zero frequency the capacitor acts as a perfect insulator and all current flows through the extracellular fluid, hence the resistance at zero frequency, $R_0$, equals $R_e$. At infinite frequency the capacitor acts as a perfect conductor and the current passes through the parallel resistive combination. The resistance at infinite frequency is given by $R_\infty = R_i R_e/(R_i+R_e)$.

Accordingly, the impedance of the equivalent circuit of Figure 3 at an angular frequency $\omega$, where $\omega=2\pi*$frequency, is given by:

$$Z = R_\infty + \frac{R_0 - R_\infty}{1+(j\omega\tau)} \qquad (1)$$

where:

    $R_\infty$ = impedance at infinite applied frequency = $R_i R_e/(R_i+R_e)$,
    $R_0$ = impedance at zero applied frequency = $R_e$ and,
    $\tau$ is the time constant of the capacitive circuit.

However, the above represents an idealised situation which does not take into account the fact that the cell membrane is an imperfect capacitor. Taking this into account leads to a modified model in which:

$$Z = R_\infty + \frac{R_0 - R_\infty}{1+(j\omega\tau)^{(1-\alpha)}} \qquad (2)$$

where $\alpha$ has a value between 0 and 1 and can be thought of as an indicator of the deviation of a real system from the ideal model.

The impedance response can be represented by a "Wessel" plot (also referred to as a Cole model or Cole-Cole plot), which is a plot of the vector sum of the resistance R and reactance $X$ that sum to impedance Z. An example Wessel plot is shown in Figure 4.

The Wessel plot is often used in BIS (Bioimpedance Spectroscopy) Bioimpedance Spectroscopy (BIS) devices, which perform multiple measurements over a range of frequencies, such as from 4 kHz to 1000 kHz, using 256 or more different frequencies within this range. The measured impedance data are used to generate a Cole plot, similar to that shown in Figure 4. A regression procedure is then used to fit the measured data to the theoretical semi-circular locus, allowing values for $R_\infty$ and $R_0$.

The regression analysis is computationally expensive, requiring a device with significant processing power to perform the calculations, which in turn results in relatively high power usage by the apparatus, requiring a larger battery, and adding to the weight and size of the apparatus.

A further issue is that a large number of data points a required to perform the regression analysis, and as measurements are typically performed at each frequency sequentially, the measurement process takes a significant amount of time, such as several seconds. This is undesirable as remaining still for long periods of time can cause discomfort for the subject. Additionally, the subject may move during the measurement procedure, which can affect the measured impedance values, for example due to changes in capacitive and/or inductive coupling between the subject and environment, leads and electrodes. This can lead to inaccuracies in the measured values.

A circle may be described by the equation:

$$(x - i)^2 + (y-j)^2 = r^2 \qquad (3)$$

where: i and j are the centre of the circle and r is the radius.

Additionally, a circle may be uniquely defined by the co-ordinates of three points $(x_{1-3}, y_{1-3})$ located on the locus, as shown in Figure 4. Accordingly, three simultaneous equations can be defined, one for each of three loci that describe the circle that fits these points, as shown by equations (4) below.

$$(x_1-i)^2 + (y_1 - j)^2 = r^2$$
$$(x_2-i)^2 + (y_2 - j)^2 = r^2 \qquad (4)$$
$$(x_3-i)^2 + (y_3 - j)^2 = r^2$$

Solving these three simultaneous equations allows calculation of the radius (r) and the co-ordinates of the centre of the circle (i, j). From these data, $R_0$ and $R_\infty$ are readily computed from geometric first principles.

Accordingly, this techniques allows a value for $R_0$ and optionally $R_\infty$ to be derived in a computationally less expensive manner than if a regression analysis is performed. Additionally, this also requires a reduced number of data points. This allows a value of $R_0$ to be determined more rapidly, and with a more basic processor than can be achieved using BIS and regression analysis, which in turn renders the device required to determine a value of $R_0$ less expensive to manufacture.

One potential disadvantage of the use of simultaneous equations is that if one of the impedance measurements is inaccurate for any reason, this can lead to a large deviation in the calculated value of $R_0$. Accordingly, in one example, impedance measurements are performed at more than three frequencies, with circle parameters for all possible combinations of impedance measurements at three frequencies being calculated. The average can be provided along with the standard deviation as a measure of the goodness of fit of the data to the Cole model. In the event that one of the measurements is inaccurate, this can be accounted for by excluding one or more outlier measurements, such as measurements that deviates the greatest amount from the mean, or measurements differing by more than a set number of standard deviations from the mean, allowing the mean to be recalculated, thereby providing more accurate values.

Whilst this process uses additional measurements, such as four or five measurements, this is still significantly less than the 256 or more frequencies typically performed using a BIS measurement protocol, allowing the measurement process to be performed more quickly.

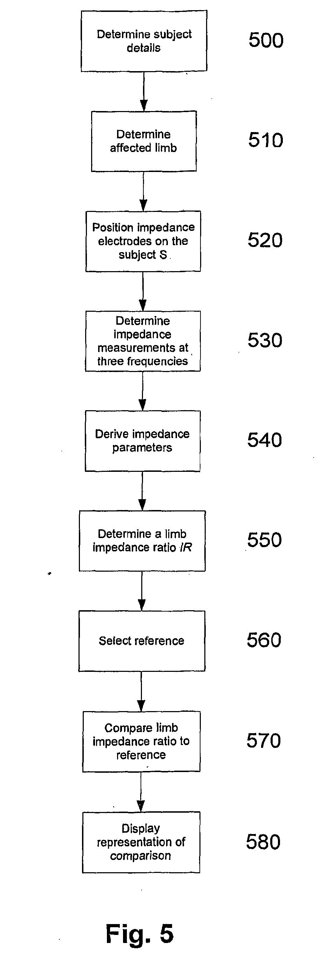

An example of the process for determining a fluid level indicator that can be used for example for diagnosing unilateral limb oedema will now be described in more detail with reference to Figure 5.

In this example, at step 500 subject details are optionally determined and provided to the processing system 102. The subject details will typically include information such as limb dominance, details of any medical interventions, as well as information regarding the subject as the subject's age, weight, height, sex, ethnicity or the like. The subject details can be used in selecting a suitable reference normal population, as will be described in more detail below.

It will be appreciated that the subject details may be supplied to the processing system 102 via appropriate input means, such as the I/O device 105. Thus, each time a subject measurement is performed this information can be input into the measuring device 100. However, more typically the information is input a single time and stored in an appropriate database, or the like, which may be connected as a peripheral device 104 via the external interface 103. The database can include subject data representing the subject details, together with information regarding previous oedema indicators, baseline measurements or impedance measurements recorded for the subject.

At step 510 the affected limb, or "at risk" limb, is determined. If it is unsure whether a particular limb is at risk, the dominant limb can alternatively be indicated as the affected limb as this will tend to have different ECF levels to the non-dominant limb. A limb can be designated in any one of a number of ways depending on the preferred implementation. Thus, for example, the affected limb can be indicated through the use of appropriate input means, such as the I/O device 105. Alternatively this information can be derived directly from the subject details, which may include an indication of the affected limb, or details of any medical interventions performed, which are in turn indicative of the affected limb.

Additionally, if the subject has, or is suspected of having, bilateral oedema, then in this instance an alternative body segment, such as a leg may be used as the unaffected limb, and this may be indicated to allow a different reference to be selected.

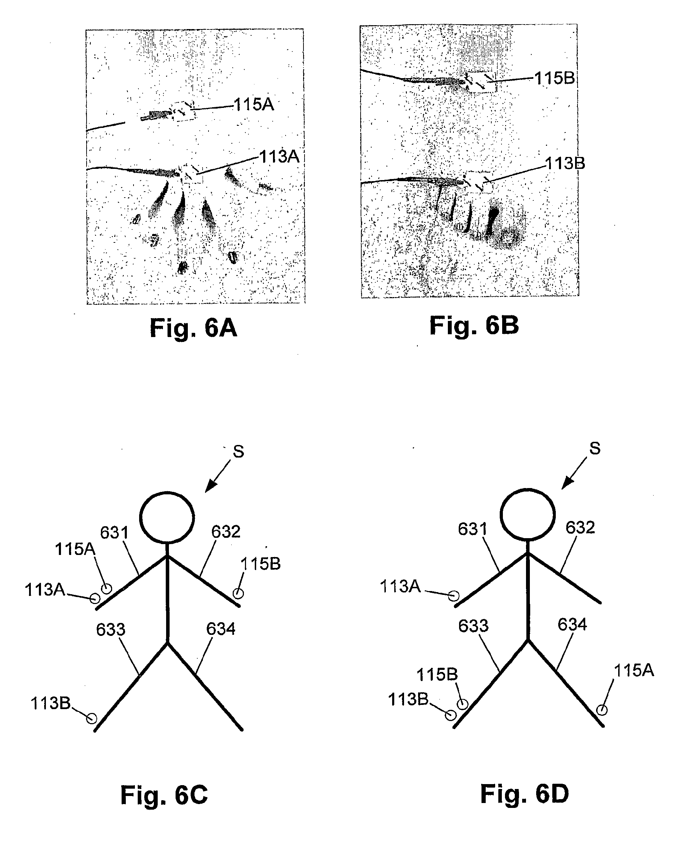

At step 520 an operator positions the electrodes 113A, 113B, 115A, 115B on the subject S, and connects the leads 123A, 123B, 125A, 125B, to allow the impedance measurements to be performed. The general arrangement is to provide electrodes on the hand at the base of the knuckles and between the bony protuberances of the wrist, as shown in Figure 6A, and on the feet at the base of the toes and at the front of the ankle, as shown in Figure 6B. The configurations shown in Figures 6C and 6D allow the right arm 631 and the right leg 633 to be measured respectively, and it will be appreciated that equivalent arrangements can be used to measure the impedance of the left leg and left arm.

It will be appreciated that this configuration uses the theory of equal potentials, allowing the electrode positions to provide reproducible results for impedance measurements. For example when current is injected between the drive electrodes 113A, 113B in Figure 6C, the electrode 115B could be placed anywhere along the left arm 632, since the whole arm is at an equal potential.

This is advantageous as it greatly reduces the variations in measurements caused by poor placement of the electrodes by the operator. It also greatly reduces the number of electrodes required to perform segmental body measurements, as well as allowing the limited connections shown to be used to measure each of limbs separately.

However, it will be appreciated that any suitable electrode and lead arrangement may be used.

In this example, at step 530 the impedance of the affected and contralateral limbs is measured at a number of frequencies. This is achieved by applying one or more current signals to the subject and then measuring the corresponding voltages induced across the subject S. It will be appreciated that in practice the signal generators 117A, 117B and the sensors 118A, 118B, return signals to the processing system 102 indicative of the current flow resulting from the applied signal, and the measured voltage signals, allowing impedance parameter values to be determined.

Following this a limb impedance ratio IR is determined. The impedance ratio is based on impedance parameter values, such as values of the impedance at zero, characteristic or infinite frequencies ($R_0$, $Z_c$, $R_\infty$). Accordingly, at step 540, these values can be derived based on the impedance response of the subject, using the simultaneous equations, as described above.

The use of an impedance ratio is desirable as this accounts for overall changes in fluid levels, which would generally lead to a relatively constant increase or decrease in the measurements for each limb, thereby reducing the likelihood of an overall fluid level change being incorrectly interpreted as a fluid level change in one limb only.

Oedema results in an increase in extracellular fluid levels in the affected limb. As the impedance parameter value $R_0$ is generally indicative of extra-cellular fluid levels, this can be used to derive a fluid level indicator that is in turn indicative of the presence, absence or degree of oedema at step 550, as shown in equation (5):

$$IR = \frac{R_0 ul}{R_0 al} \qquad\qquad , \qquad (5)$$

where: IR is the impedance ratio representing the fluid level

$R_0 ul$ is the impedance of the unaffected limb at zero frequency

$R_0 al$ is the impedance of the affected limb at zero frequency

However, other impedance parameters can be used. For example, the impedance ratio can be determined by calculating an index based on a ratio of the ECF and ICF levels for a respective limb, with the impedance ratio being determined based on a ratio of the index for the affected limb to the unaffected limb. The index (I) can be calculated using the equation (6):

$$I = \frac{R_\infty}{R_0 - R_\infty} \qquad (6)$$

In this example, the fluid level indicator is given by:

$$IR = \frac{I_{affected}}{I_{unaffected}} \qquad (7)$$

At step 560 a reference is selected. The reference is typically derived from equivalent measurements made on a normal population (subject's not suffering from oedema) that is relevant to the subject under study. Thus, the normal population is typically selected taking into account factors such as medical interventions performed, ethnicity, sex, height, weight, limb dominance, the affected limb, or the like.

Therefore if the test subject has unilateral lymphoedema of the dominant arm and is female then the normalised data drawn from the normal population database will be calculated from the dominant arm impedance ratio measurements from female subjects that are present in the normal population database. If the subject has bilateral arm oedema, then typically the unaffected limb is taken to be one of the subject's legs, and a reference based on a ratio of an arm to a leg in the normal population is used.

Accordingly, at this stage the processing system 102 typically accesses reference populations stored in the database, or the like. This may be performed automatically by the processing system 102 using the subject details. Thus for example, the database may include a look-up table that specifies the normal population that should be used given a particular set of subject details. Alternatively selection may be achieved in accordance with predetermined rules that can be derived using heuristic algorithms based on selections made by medically qualified operators during previous procedures. Alternatively, this may be achieved under control of the operator, depending on the preferred implementation.

It will be appreciated by persons skilled in the art that operators may have their own reference stored locally. However, in the event that suitable references are not available, the processing system 102 can be used to retrieve a reference from a central repository, for example via an appropriate server arrangement. In one example, this may be performed on a pay per use basis.

Alternatively, in the event that a suitable reference is not available predetermined standard reference values may be used.

In one example, the reference values are based on a comparison of the impedance ratio to a normal range of impedance ratios established in a healthy population. Patient's whose impedance ratio is greater than 3 standard deviations away from the mean ratio are defined as having lymphoedema.

A slight and known effect on the impedance ratio is induced by limb dominance; therefore there are two sets of normal impedance ratios for the arms of women for example. One when the dominant arm is defined as the at-risk arm and one when the non-dominant arm is defined as the at-risk arm Example population mean and standard deviations for female subjects are set out in table 1 below.

Table 1

| standard deviation | at-risk arm dominant | at-risk arm non dominant |
|---|---|---|
| $-3\sigma$ | 0.935 | 0.862 |
| $-2\sigma$ | 0.969 | 0.896 |
| $-1\sigma$ | 1.003 | 0.930 |
| $\mu$ | 1.037 | 0.964 |
| $+1\sigma$ | 1.071 | 0.998 |
| $+2\sigma$ | 1.105 | 1.032 |
| $+3\sigma$ | 1.139 | 1.066 |

In one example, the reference values are based on the mean impedance ratio, and an impedance ratio value three standard deviations from the mean impedance ratio for the normal population, and example values are set out below. However it will be appreciated that different values can be used as appropriate and that these values are for illustration only:

$\mu$ = 1.037
$3\sigma$ = 1.139

As an alternative to using different references based on the limb dominance, gender, or the like, a further variation is for the impedance ratio to be modified using a correcting factor to take into account differences in fluid levels caused by limb dominance, or the need to measure impedances of dissimilar limbs. In this instance, the fluid level indicator based on the impedance ratio is calculated, and then modified using the correcting factor, allowing the modified fluid level indicator to be compared to default reference values. The correcting factor may be established by surveying subjects unaffected by lymphoedema, and will typically be based on mean values for the population.

It will be appreciated that establishing a respective correction factor for each particular body segment combination that may be used allows a common reference to be used for performing any subsequent analysis of fluid levels. Alternatively, however, differences in fluid levels within different body segments or limbs can be accounted for by selecting a respective reference for the particular limb or body segment combination being measured.

At step 570 the fluid level indicator in the form of the impedance ratio, or a corrected impedance ratio, can be compared to a threshold based on the reference, with an indication of the result of the comparison being displayed at step 580. In one example, this can be achieved by displaying the fluid level indicator, such as the impedance ratio, together with any threshold based on the reference, thereby allowing visual comparison. Alternatively, the result of the comparison can be used to determine an oedema indicator used in assessing the presence, absence or degree of oedema.

In one example, an oedema indicator is determined by scaling the impedance ratio using the reference population, and in particular using the mean and the standard deviation of the reference. This can be performed so that the presence of oedema is indicated by a memorable value. To achieve this, in one example, the transformation of the impedance ratio to an oedema indicator value is governed by the following formula:

$$L - Dex = \frac{sf \times (IR - \mu)}{3\sigma - \mu} \qquad (8)$$

where: *L-Dex* is the oedema indicator
*IR* is the impedance ratio
$\mu$ is the mean impedance ratio for a reference population
$3\sigma$ is impedance ratio value that is three standard deviations from the mean population impedance ratio value
*sf* is the scaling factor

The scaling factor is selected so that the thresholds correspond to a memorable value, and in particular, the scaling factor is typically an integer value, and more typically a multiple of ten. Thus, in one example, the scaling factor is set to a value of "10", so that the threshold occurs at "10". As a result, an oedema indicator value of greater than "10" is indicative of oedema, whilst a value of below "10" is used to indicate an absence of oedema.

For example, if a subject S whose at-risk arm is the dominant arm and has an impedance ratio of 1.207, the subject's impedance ratio is scaled using a suitable normal population. For the purpose of this example, the normal population has a mean impedance ratio value of 1.037 and a three standard deviation value of 1.139. This leads to an oedema indicator of:

$$L\text{-}Dex = (1.207\text{-}1.037)\text{x}10/(1.139\text{-}1.037) = 16.6$$

The fluid level indicator, and optionally the oedema indicator can be stored together with any relevant information, such as the time and date on which the measurement was performed, details of the operator of the measuring device 100, or the like. This allows for the measured oedema indicator to be subsequently retrieved and used in tracking the development and/or progression of the oedema, allowing the effectiveness or need for treatment to be evaluated.

Display of the representation may be achieved in a number of ways, such as by presenting the representation on a suitable display, for example, using the I/O device 105, or alternatively by providing the representation in a hard copy form using an appropriate printer, although any suitable technique may be used.

A comparison of the above described technique simultaneous equation technique versus the regression analysis of BIS was performed using groups of subjects, characteristics of which are outlined in Table 1 below.

Table 2

|  | Group A | Group B | *Group C* |
|---|---|---|---|
| No. of subjects | 46 | 66 | *45* |
| Male:female | 14:32 | 39:27 | *24:21* |
| Age (y) | $33.1 \pm 9.7$ | $37.8 \pm 7.1$ | *$39.1 \pm 5.3$* |
| Weight (kg) | $63.6 \pm 9.3$ | $82.4 \pm 9.0$ | *$102.6 \pm 13.1$* |
| Height (cm) | $169.1 \pm 8.9$ | $172.5 \pm 9.6$ | *$174.1 \pm 9.0$* |
| Fat-free mass (kg) | $47.7 \pm 9.0$ | $56.1 \pm 11.0$ | *$61.4 \pm 11.4$* |
| *BMI ($kg/m^2$)* | *$22.1 \pm 2.0$* | *$27.7 \pm 1.5$* | *$33.7 \pm 3.0$* |

Where, BMI is the body mass index and the Fat-free mass assessed by dual X-ray absorptiometry (DXA).

Whole-body, wrist-to-ankle, impedance was measured using the tetrapolar electrode arrangement shown in Figures 6C and 6D, technique while the subjects had been lying prone for the duration of the DXA scan (20 min). Impedance measurements data were collected at 496 discrete logarithmically spaced frequencies in the range of 4 to 1024 kHz. Impedance (ohm) and phase angle (degrees) were recorded.

The resulting reactance and resistance values were fitted to the Cole model, with estimates for the impedance parameter values $R_0$, $R_\infty$ being determined by fitting reactance and resistance to the semicircular locus. A percent standard error of the estimate of the radius of the fitted curve was used as the goodness of fit parameter.

Additionally, resistance and reactance values for four selected frequencies were determined. In this example, this process was performed at two different sets of four frequencies, designated as MFBIA-1 and MFBIA-2, respectively. The selected frequencies were as follows:

MFBIA-1 - 14.2 kHz, 56.9 kHz, 187.5 kHz, and 679.1 kHz;
MFBIA-2 - 25 kHz, 50 kHz, 100 kHz, and 200 kHz.

Each of these four frequencies were fitted to a semicircular locus, as predicted by the Cole model, allowing the simultaneous equation techniques described above to be performed. As only three points on the circumference of a circle are required to define the circle, all possible combinations of three reactance-resistance loci from the four available pairs of data were used to produce estimates of $R_0$ and $R_\infty$.

The mean value with its associated standard error, as the index of goodness of fit, was determined.

The means and standard deviations of data for subjects in each BMI band were calculated. The different methods for the calculation of $R_0$ and $R_\infty$ were compared by concordance correlation analysis with values for individuals compared by paired t test and the limits of agreement method of Bland and Altman.

All impedance data fitted the Cole model well irrespective of the method of derivation as indicated by the measurements of goodness of fit shown in Table 2, with percent standard errors all being less than 5%. Goodness of fit was similar for all three BMI groups when parameters were estimated by conventional BIS analysis. Both MFBIA methods estimated $R_\infty$ with a greater degree of precision, as indicated by the smaller standard error for repeat determinations, than $R_0$, although for method MFBIA-1, precision worsened as BMI of the subjects increased and was generally worse than that observed for method MFBIA-2. In contrast, $R_\infty$ was estimated with greater precision by method MFBIA-1 than by method MFBIA-2.

Table 3

|  | Group A (n = 46) BMI <24.9 kg/m$^2$ | Group B (n = 66) BMI 25-29.9 kg/m$^2$ | Group C (n = 45) BMI > 30 kg/m$^2$ |
|---|---|---|---|
| MFBIA-1 (14.2, 56.9, 187.5, and 679.1 kHz) |  |  |  |
| $R_0$ | 1.46 ± 0.23 | 2.98 ± 0.43 | 3.08 ± 0.60 |
| $R_\infty$ | 0.16 ± 0.02 | 0.30 ± 0.07 | 0.23 ± 0.02 |
| MFBIA-2 (5, 50, 100 and 200 kHz) |  |  |  |
| $R_0$ | 1.11 ± 0.14 | 1.33 ± 0.17 | 1.17 ± 0.15 |
| $R_\infty$ | 0.67 ± 0.15 | 0.64 ± 0.12 | 0.51 ± 0.07 |
| BIS |  |  |  |
| Model fit | 1.52 ± 0.10 | 2.05 ± 0.17 | 1.58 ± 0.11 |

A comparison of the resulting impedance parameter values are shown in Table 4.

Table 4

|  | MFBIA-1 frequencies 14.2, 56.9, 187.5, and 679.1 kHz |  |  |  |  |
|---|---|---|---|---|---|
|  | BIS method | MFBIA-1 | P | r | Agreement (%; 2 SD) |
| Normal-weight subjects (BMI <24.9 kg/m$^2$) |  |  |  |  |  |
| $R_0(\Omega)$ | 667.1 ± 79.1 | 675.5 ± 82.5 | 0.003 | 0.970 | -6. 6 to 4.1 |
| $R_\infty(\Omega)$ | 458.3 ± 69.3 | 467.7 ± 65.4 | 0.001 | 0.975 | -7.3 to 3.2 |
| Overweight subjects (BMI25-29.9 kg/m$^2$) |  |  |  |  |  |
| $R_0(\Omega)$ | 596.4 ± 64.6 | 608.3 ± 68.2 | 0.011 | 0.946 | -7.9 to 2.8 |
| $R_\infty(\Omega)$ | 390.8 ± 53.4 | 398.7 ± 54.4 | 0.001 | 0.967 | -7.8 to 3.7 |
| Obese subjects (BMI > 30 kg/m$^2$) |  |  |  |  |  |
| $R_0(\Omega)$ | 563.3 ± 58.7 | 580.8 ± 72.1 | 0.001 | 0.914 | -10.6 to 4.4 |
| $R_\infty(\Omega)$ | 368.3 ± 47.4 | 378.0 ± 49.4 | 0.001 | 0.951 | -8.9 to 3.6 |
|  |  |  |  |  |  |
|  | MFBIA-2 frequencies 5, 50, 100, and 200 kHz |  |  |  |  |
|  | BIS method | MFBIA-2 | P | r | Agreement (%; 2 SD) |
| Normal-weight subjects (BMI <24.9 kg/m$^2$) |  |  |  |  |  |
| $R_0(\Omega)$ | 667.1 ± 79.1 | 666.0 ± 77.0 | NS | 0.986 | -3.7 to 4.0 |
| $R_\infty(\Omega)$ | 458.3 ± 69.3 | 469.4 ± 67.5 | 0.001 | 0.967 | -8.5 to 3.6 |
| Overweight subjects (BMI25-29.9 kg/m$^2$) |  |  |  |  |  |
| $R_0(\Omega)$ | 596.4 ± 64.6 | 595.0 ± 64.4 | NS | 0.971 | -4.8 to 5.3 |
| $R_\infty(\Omega)$ | 390.8 ± 53.4 | 402.2 ± 55.2 | 0.001 | 0.954 | -9.1 to 3.2 |

(continued)

| Obese subjects (BMI >30 kg/m$^2$) | | | | | |
|---|---|---|---|---|---|
| $R_0(\Omega)$ | 563.3 $\pm$ 58.7 | 561.3 $\pm$ 61.3 | NS | 0.985 | *-3.1 to 3.8* |
| $R_\infty(\Omega)$ | *368.3 $\pm$ 47.4* | *379.2 $\pm$ 50.5* | *0.001* | *0.944* | *-9.6 to 3.7* |

The above data show that all three methods for estimating $R_0$ and $R_\infty$ are highly correlated *(r_ 0.91; Table 4).*

The two methods based on MFBIA measurements generally estimated slightly higher values for $R_0$ and $R_\infty$ compared with traditional BIS, although this bias was small, averaging 2%, and was similar in magnitude for both methods.

Absolute values of $R_0$ and $R_\infty$ decreased as BMI increased and generally the correlations between the MFBIA and BIS methods worsened slightly with increasing BMI, particularly for $R_\infty$. Correlations with BIS were slightly lower for MFBIA-1 compared with MFBIA-2 for $R_0$, whereas the opposite pattern, higher correlations for MFBIA-2 with BIS, were observed for $R_\infty$

Accordingly, the optimized frequency method MFBIA-1 estimated $R_\infty$ more closely to the corresponding BIS values, whereas method MFBIA-2 was better at estimating $R_0$ compared with BIS. Correspondingly, the limits of agreement for $R_0$ between the BIS and MFBIA methods were slightly larger by method MFBIA-1 than by method MFBIA-2, whereas, for $R_\infty$, the limits of agreement were greater for method MFBIA-2. Generally, $R_0$ was estimated more accurately, based on correspondence with BIS values as the reference, than $R_\infty$

Accordingly, this demonstrates that the above described technique using the simultaneous equations provides an adequate substitution for the more extensive BIS measurement protocol, whilst allowing the method to be implemented using a more straightforward device, and with a reduced measurement time.

It is also apparent that selection of a suitable choice of frequencies of measurement can improve the resulting agreement with the BIS measurements. In particular, in the above described examples, when it is desired to calculate $R_0$ it is apparent that avoiding higher frequencies may be desirable. One reason for this is that the frequency range used more accurately represents the frequency of the impedance parameter value being calculated. Another factor is that higher frequency measurements tend to be more prone to error due to noise.

In one example, the preferred frequencies for use in calculating $R_0$ using MFBIA and simultaneous equations is are in the frequency range 10 to 500 kHz, thereby avoiding more error prone higher frequencies. In another example, four frequencies used are in the range 25 kHz to 200 kHz.

Further improvements can be achieved by taking steps to reduce the impact of errors.

In one example, this is achieved by generating the impedance ratio described above. It will be appreciated that the impedance ratio is based on the impedance parameters measured for each limb. Accordingly, if there is an inaccuracy in the calculated impedance parameter, due to the calculation used, this will tend to be similar for each measurement. Thus, for example, the calculated impedance parameter $R_0$ may be slightly increased as compared to a more accurate value determined for example using BIS. However, if there is an increase for each limb, or limb segment, then the relative magnitude of this error will be reduced when the impedance ratio is calculated, thereby overcoming the inaccuracies to a large degree.

In this regard, the accuracy of the measurement of impedance can be subject to a number of external factors. These can include, for example, the effect of capacitive coupling between the subject and the surrounding environment, the leads and the subject, the electrodes, or the like, which will vary based on factors such as lead construction, lead configuration, subject position, or the like. Additionally, there are typically variations in the impedance of the electrical connection between the electrode surface and the skin (known as the "electrode impedance"), which can depend on factors such as skin moisture levels, melatonin levels, or the like. A further source of error is the presence of inductive coupling between different electrical conductors within the leads, or between the leads themselves.

Such external factors can lead to inaccuracies in the measurement process and subsequent analysis and accordingly, it is desirable to be able to reduce the impact of external factors on the measurement process.

One form of inaccuracy that can arise is caused by the voltages across the subject being unsymmetrical, a situation referred to as an "imbalance". Such a situation results in a significant signal voltage at the subject's body centre, which in turn results in stray currents arising from parasitic capacitances between the subject's torso and the support surface on which the subject is provided.

The presence of an imbalance, where the voltage across the subject is not symmetrical with respect to the effective centre of the subject, leads to a "common mode" signal, which is effectively a measure of the signal at the subject S that is unrelated to the subject's impedance.

To help reduce this effect, it is therefore desirable for signals to be applied to the subject S that they result in a symmetrical voltage about the subject's body centre. As a result, a reference voltage within the subject S, which is equal to a reference voltage of the measurement apparatus, will be close to the effective body centre of the subject, as considered relative to the electrode placement. As the measuring device reference voltage is typically ground, this results in the body centre of the subject S being as close to ground as possible, which minimises the overall signal magnitude across the subject's torso, thereby minimising stray currents.

In one example, a symmetrical voltage about the sensing electrodes can be achieved by using a symmetrical voltage source, such as a differential bidirectional voltage drive scheme, which applies a symmetrical voltage to each of the drive electrodes 113A, 113B. However, this is not always effective if the contact impedances for the two drive electrodes 113A, 113B are unmatched, or if the impedance of the subject S varies along the length of the subject S, which is typical in a practical environment.

In one example, the apparatus overcomes this by adjusting the differential voltage drive signals applied to each of the drive electrodes 113A, 113B, to compensate for the different electrode impedances, and thereby restore the desired symmetry of the voltages across the subject S. This process is referred to herein as *balancing* and in one example, helps reduce the magnitude of the common mode signal, and hence reduce current losses caused by parasitic capacitances associated with the subject.

The degree of imbalance, and hence the amount of balancing required, can be determined by monitoring the signals at the sense electrodes 115A, 115B, and then using these signals to control the signal applied to the subject via the drive electrodes 113A, 113B. In particular, the degree of imbalance can be calculated by determining an additive voltage from the voltages detected at the sense electrodes 115A, 115B.

In one example process, the voltages sensed at each of the sense electrodes 115A, 115B are used to calculate a first voltage, which is achieved by combining or adding the measured voltages. Thus, the first voltage can be an additive voltage (commonly referred to as a common mode voltage or signal) which can be determined using a differential amplifier.

In this regard, a differential amplifier is typically used to combine two sensed voltage signals $V_a$, $V_b$, to determine a second voltage, which in one example is a voltage differential $V_a$-$V_b$ across the points of interest on the subject S. The voltage differential is used in conjunction with a measurement of the current flow through the subject to derive impedance values. However, differential amplifiers typically also provide a "common mode" signal $(V_a+V_b)/2$, which is a measure of the common mode signal.

Whilst differential amplifiers include a common mode rejection capability, this is generally of only finite effect and typically reduces in effectiveness at higher frequencies, so a large common mode signal will produce an error signal superimposed on the differential signal.

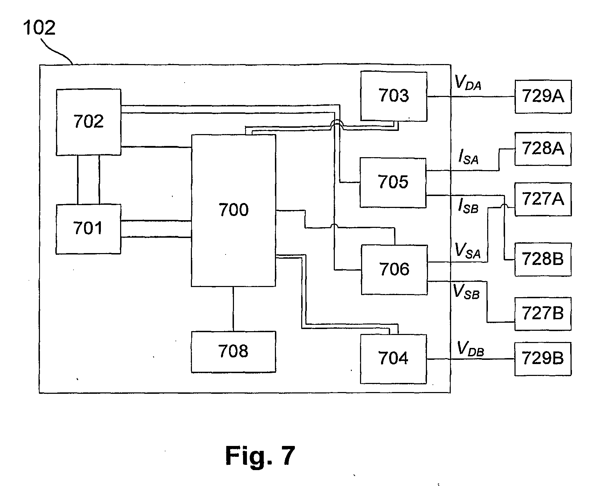

A specific example of the functionality implemented by the processing system 102 will now be described with reference to Figure 7. In this example the processing system 102 implements the functionality using appropriate software control, via software stored in a memory, although any suitable mechanism may be used.

In this example the processing system 102 includes a timing and control module 700, an interface module 701, an analysis module 702, sine wave look up tables (LUTs) 703, 704, a current module 705, and a voltage module 706.

A number of analogue to digital converters (ADCs) 727A, 727B, 728A, 728B and digital to analogue converters (DACs) 729A, 729B are provided for coupling the processing system 102 to the sensors 118A, 118B and the signal generators 117A, 117B, as will be described in more detail below.

In use, the processing system 102 determines the frequency and amplitude of signals to be applied to the subject S. The timing and control module 700 typically receives this information in accordance with input commands received from the input 105 via the interface module 701 and uses this information to access the LUTs 703, 704, which in turn cause a digital sine wave signal to be produced based on the specified frequency and amplitude. The digital control signals are transferred to the DAC's 729A, 729B, to thereby allow analogue control signals indicative of the voltage drive signals $V_{DA}$, $V_{DB}$ to be produced.

Measured analogue voltage and current signals $V_{SA}$, $V_{SB}$, $I_{SA}$, $I_{SB}$ are digitised by the ADC's 727, 728 and provided to the current and voltage modules 705, 706. This allows the processing system 102 to determine the current flow by having the current module 705 determine the total current flow through the subject using the two current signals $I_{SA}$, $I_{SB}$, with an indication of this being provided to the analysis module 702. The voltage module 706, which is typically in the form of a differential voltage amplifier, or the like, operates to determine a differential voltage, which is also transferred to the analysis module 702, allowing the analysis module to determine impedance values using the current and differential voltage signals.

In addition to this, the voltage module 706 determines a common mode signal, which is returned to the timing and control module 700. This allows the timing and control module 700 to determine any imbalance in the voltage sensed at the subject S, which as mentioned above is indicative of the reference voltage not being positioned centrally within the subject S, with respect to the electrodes.

If the degree of imbalance is unacceptable the timing and control module 700 can adjust the relative amplitude and/or phase of the sine waves representing the voltage drive signals $V_{DA}$, $V_{DB}$ as will be described below, allowing a new differential voltage, hence indication of any imbalance, to be determined.

Once the imbalance is determined to be acceptable the timing and control module 700 can provide an indication of this to the analysis module 702, allowing this to use appropriate analysis, such as phase quadrature extraction, to determine a ratio and phase difference for the measured impedance, based on the current flow through the subject and the differential voltage signals. The ratio and phase can then be used by to determine reactance and resistance parameter values and then calculate the fluid level indicator, which can be transferred to a display via the interface module 701.

The control module 700 may also be coupled to a fault detection module 708. This monitors the magnitude of signals applied to the subject to determine if these are within acceptable threshold levels. If not, the fault detection module 708 can cause the process to be halted or to allow an alert to be generated.

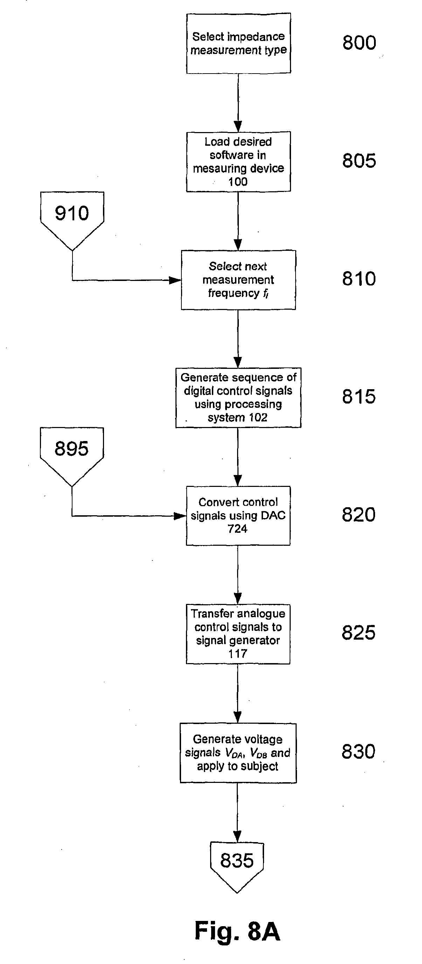

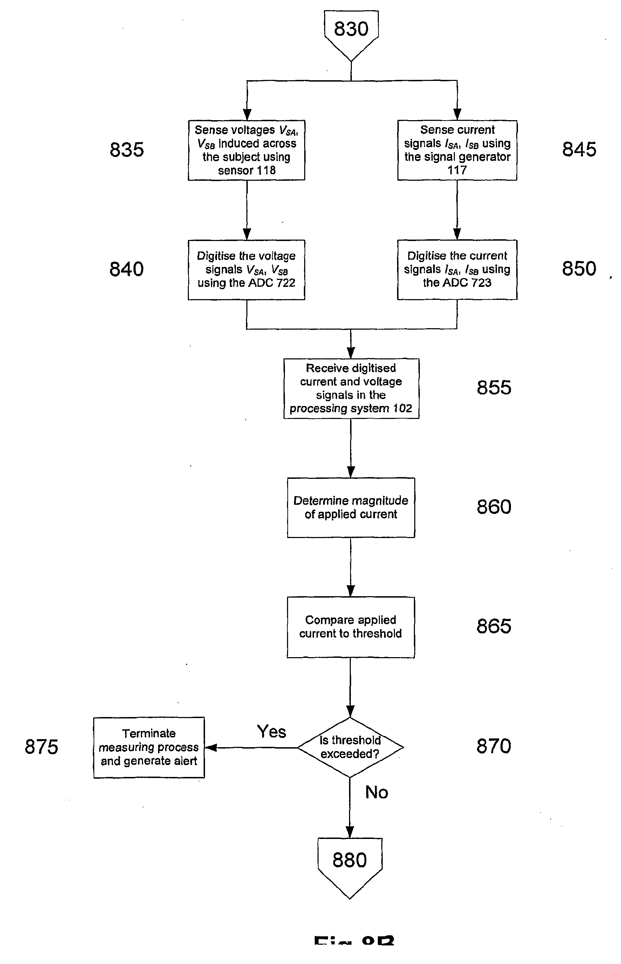

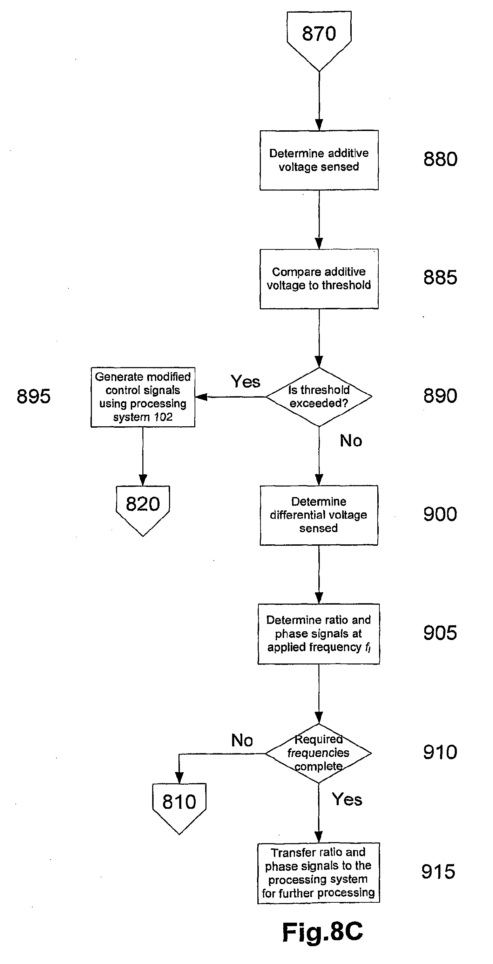

An example of the process for performing impedance measurements will now be described with reference to Figures 8A to 8C.

At step 800 an impedance measurement type is selected. At step 810 the processing system 102 selects a next measurement frequency $f_i$, allowing a sequence of digital voltage control signals at step 815, as described above. The digital control signals are converted to analogue control signals indicative of the voltage drive signals $V_{DA}$, $V_{DB}$ using the DACs 729A, 729B at step 820. This allows the analogue control signals to be provided to each of the signal generators 117A, 117B at step 825, causing each signal generator 117A, 117B to generate respective voltage drive signals $V_{DA}$, $V_{DB}$ and apply these to the subject S at step 830, via the respective drive electrodes 113A, 113B.

At step 835 the voltage induced across the subject is determined by having the sensors 118A, 118B sense voltages $V_{SA}$, $V_{SB}$ at the sense electrodes, 115A, 115B, with the sensed voltage signals $V_{SA}$, $V_{SB}$ being digitised by the corresponding ADC 727A, 727B at step 840. At step 845 current signals $I_{SA}$, $I_{SB}$, caused by application of the voltage drive signals $V_{DA}$, $V_{DB}$, are determined using the signal generators 117A, 117B. An indication of the current signals $I_{SA}$, $I_{SB}$ are transferred to the ADCs 728A, 728B for digitisation at step 850.

At step 855 the digitised current and voltage signals $I_{SA}$, $I_{SB}$, $V_{SA}$, $V_{SB}$ are received by the processing system 102 allowing the processing system 102 to determine the magnitude of the applied current $I_S$ at step 860. This may be performed using the current addition module 705 in the above described functional example of Figure 7, allowing the fault detection module 708 to compare the total current flow Is through the subject to a threshold at step 865. If it is determined that the threshold has been exceeded at step 870 then the process may terminate with an alert being generated at step 875.

This situation may arise, for example, if the device is functioning incorrectly, or there is a problem with connections of electrodes to the subject, such as if one is not in correct electrical contact with the subject's skin. Accordingly, the alert can be used to trigger a device operator to check the electrode connections and/or device operation to allow any problems to be overcome. It will be appreciated, that any suitable form of corrective action may be taken such as attempting to restart the measurement process, reconnecting the electrodes to the subject S, reducing the magnitude of the current through the subject, or the like.

At step 880 the processing system 102 operates to determine a common mode voltage based on the amplitude of the sensed voltages $V_{SA}$, $V_{SB}$ sensed at each of the electrodes 115A, 115B, and this is typically achieved using the voltage processing module 706 in the above functional example. The common mode voltage or common mode signal is then used to determine any imbalance at step 885.

At step 890 an assessment is made as to whether the imbalance is acceptable. This may be achieved in any one of a number of ways, such as by comparing the amplitude of the common mode signal to a threshold, or the like. The threshold will generally be previously determined and stored in memory for example during device manufacture or calibration.

In the event that the imbalance is deemed to not be acceptable, then at step 895 the processing system 102 modifies the digital control signals representing the voltage drive signals $V_{DA}$, $V_{DB}$ to reduce the imbalance. This is typically achieved by having the processing system 102 implement an algorithm that adjusts the applied voltage drive signals $V_{DA}$, $V_{DB}$ to maintain the common mode voltage at the centre of the body as close to the device reference voltage as possible. This is generally achieved by adjusting the amplitude and/or phase of the voltage drive signals $V_{DA}$, $V_{DB}$ applied to the subject, using the algorithm. The nature of this adjustment will depend on the nature of the imbalance, and an example algorithm will be described in more detail below.

The process can then return to step 820 to allow the modified digital control signals to be converted to analogue signals using DACs 724, with modified voltage drive signals $V_{DA}$, $V_{DB}$ being applied to the drive electrodes 113A, 113B. This process is repeated until an acceptable balance is achieved.

Once an acceptable balance is achieved, the processing system 102 operates to determine the differential voltage sensed across the subject at step 900. In the functional example described above with respect to Figure 7, this can be achieved using the differential voltage module 706. At step 905 the analysis module 702 operates to determine ratio and phase signals, representing the impedance of the subject S, at the applied frequency $f_i$ using the current and differential voltage signals. In the above functional example, this can be performed using the analysis module, and some form of signal analysis, such as phase quadrature analysis, depending on the preferred implementation.

At step 910, it is determined if measurements at each of the frequencies have been performed, and if not, the process may return to step 810 to allow the process to be repeated at a next measurement frequency $f_i$. Otherwise if all required frequencies are complete, the measurement process can terminate, allowing the processing system 102 to analyse the impedance measurements, and determine the fluid level indicator, as described above.

Accordingly, it will be appreciated that by repeating the above described process this allows a number of impedance measurements to be performed over three or four frequencies. Furthermore, prior to at least one, and more typically, to each measurement, a check can be performed to ensure that the common mode of the subject and the device are approximately matched, thereby reducing inaccuracies in the measurement procedure.

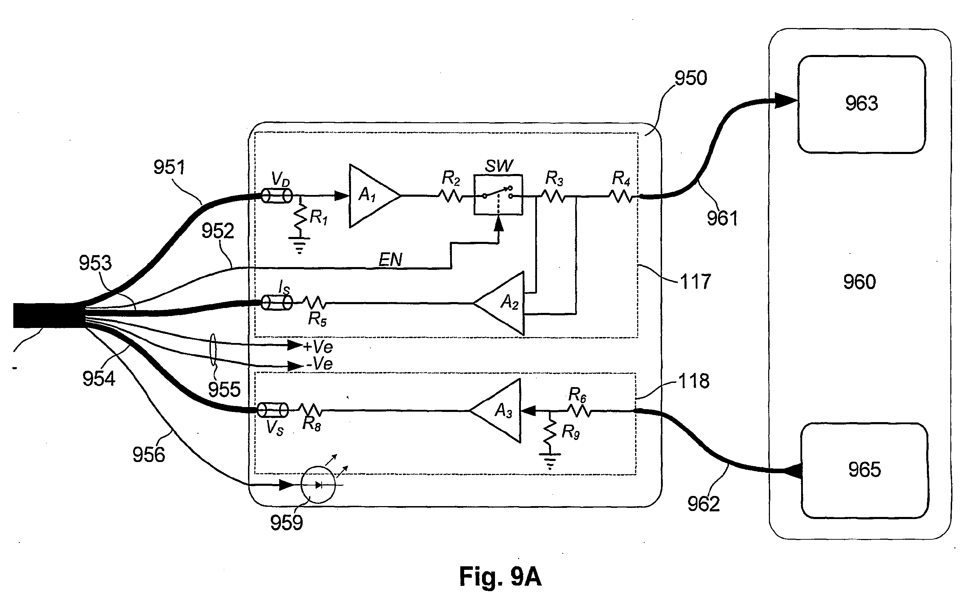

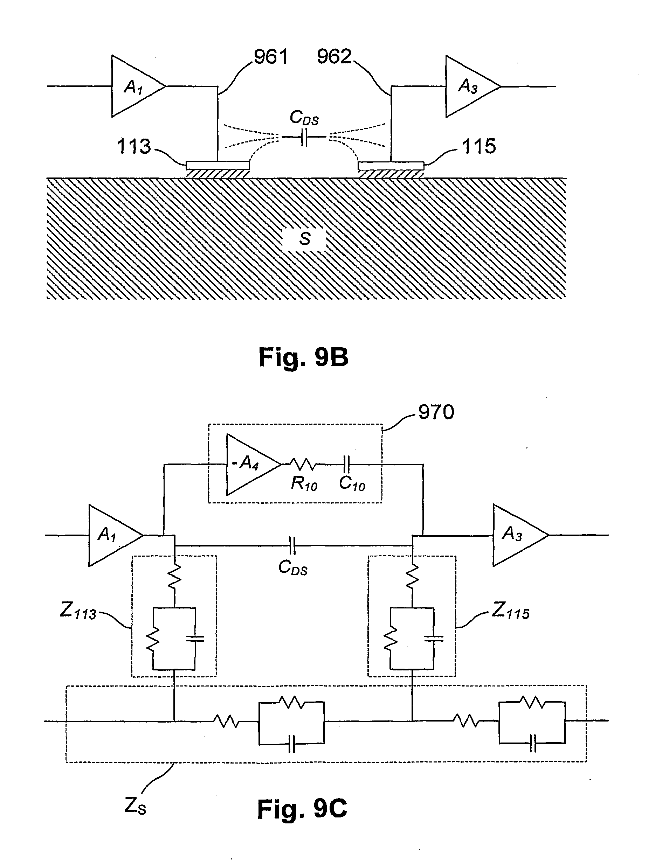

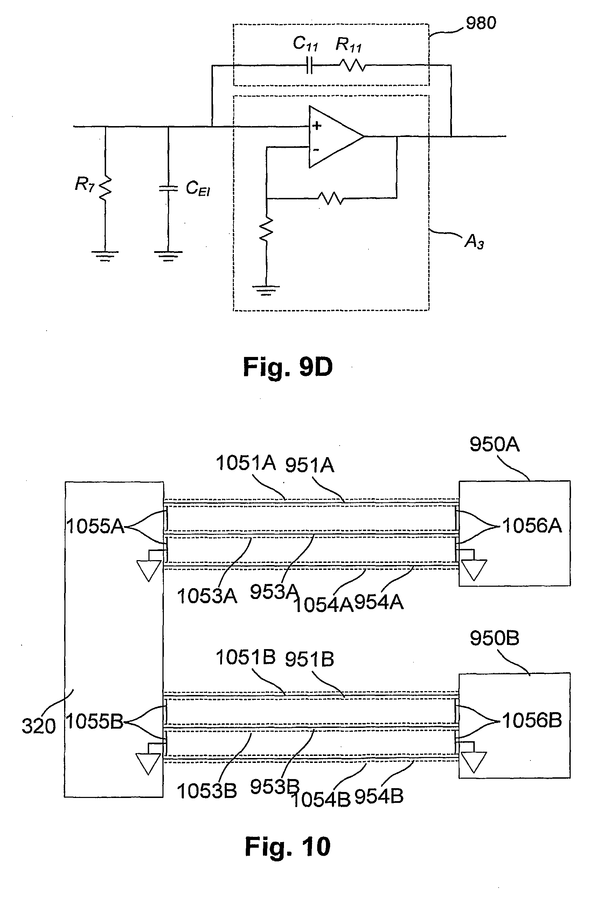

An example of an electrode system for a single one of the channels, which incorporates both a drive electrode 113 and sense electrode 115, will now be described with reference to Figure 9.

The electrode system incorporates a first substrate 950, such as a printed circuit board (PCB), or the like, having the respective signal generator 117 and sensor 118 mounted thereon. The general functionality of the signal generator 117 and sensor 118 are represented by the components shown. In practice a greater number of components may be used in a suitable arrangement, as would be appreciated by persons skilled in the art, and the components shown are merely intended to indicate the functionality of the signal generator and the sensor 117, 118.

The substrate 950 and associated components may be provided in a suitable housing to protect them during use, as will be appreciated by persons skilled in the art.

The signal generator 117 and the sensor 118 are coupled via respective cables 961, 962 to conductive pads 963, 965, which may be mounted on a second substrate 960, and which form the first and second electrodes 113, 115, respectively. It will be appreciated that in use, the cables 961, 962 may include clips or the like, to allow the conductive pads to be easily replaced after use.

As will be appreciated, the conductive pads are typically formed from a silver pad, having a conductive gel, such as silver/silver chloride gel, thereon. This ensures good electrical contact with the subject S.

The conductive pads may be mounted on the substrate 960, so as to ensure that the conductive pads 963, 965 are positioned a set distance apart in use, which can help ensure measurement consistency. Alternatively the conductive pads 963, 965 can be provided as separate disposable conductive pads, coupled to the first substrate 950 by cables 961, 962. Other suitable arrangements may also be used.

In one example, the substrate 960 is formed from a material that has a low coefficient of friction and/or is resilient, and/or has curved edges to thereby reduce the chances of injury when the electrodes are coupled to the subject. The substrate 960 is also typically arranged to facilitate electrical contact between the conductive pads 963, 965 and the subject's skin at the typical measurement sites, such as the wrist and ankle. This can be achieved by providing a substrate 960 that adapts to, or is shaped to conform with the irregular shapes and angles of the anatomy.

In this example, the signal generator 117 includes an amplifier $A_1$ having an input coupled to a cable 951. The input is also coupled to a reference voltage, such as ground, via a resistor $R_1$. An output of the amplifier $A_1$ is connected via a resistor $R_2$, to a switch *SW*, which is typically a CMOS (complementary metal-oxide semiconductor) switch or a relay that is used to enable the voltage source. The switch *SW* is controlled via enabling signals *EN* received from the processing system 102 via a cable 952.

The switch *SW* is in turn coupled via two resistors $R_3$, $R_4$, arranged in series, and then, via the cable 961, to the conductive pad 963. A second amplifier $A_2$ is provided with inputs in parallel with the first of the two series resistor $R_3$ and with an output coupled via a resistor $R_5$, to a cable 953.

It will be appreciated from the above that the cables 951, 952, 953 therefore forms the lead 123 of Figure 1. A range of different resistor values may be used, but in one example, the resistors have values of $R_1 = R_2 = R_5 = 50\Omega$, and $R_3 = R_4 = 100\Omega$.

The sensor 118 generally includes an amplifier $A_3$ having an input connected via a resistor $R_6$, to the cable 962. The input is also coupled via a resistor $R_7$, to a reference voltage such as a ground. An output of the amplifier $A_3$ is coupled to a cable 954, via a resistor $R_7$.

It will be appreciated from the above that the cable 954 therefore forms the lead 125 of Figure 1. A range of different resistor values may be used, but in one example, the resistors have values of $R_6 = 100\Omega$, $R_7 = 10M\Omega$ and, $R_8 = 50\Omega$.

Optional power cables 955 can be provided for supplying power signals + *Ve, - Ve,* for powering the signal generator 117 and the sensor 118, although alternatively an on board power source such as a battery, may be used. Additionally, a cable 956 may be provided to allow an LED 957 to be provided on the substrate 950. This can be controlled by the processing system 102, allowing the operating status of the electrode system to be indicated.

Operation of the signal generator 117 and the sensor 118 will now be described in more detail. For the purpose of this explanation, the voltage drive signal, current signal and sensed voltage will be generally indicated as $V_D$, $I_S$, $V_S$, and in practice, these would be equivalent to respective ones of the voltage drive signals, current signals and sensed voltages $V_{DA}$, $V_{DB}$, $I_{SA}$, $I_{SB}$, $V_{SA}$, $V_{SB}$ in the example above.

In use, the amplifier $A_1$ operates to amplify the analogue voltage signal received from the DAC 729 and apply this to the subject S via the cable 961, so that the applied voltage drive signal $V_D$ drives a current signal $I_S$ through the subject S. The voltage drive signal $V_D$, will only be applied if the switch *SW* is in a closed position and the switch *SW* can therefore be placed in an open position to isolate the voltage source from the subject S. This may be used if a pair of drive and sense electrodes 113, 115 are being used to sense voltages only, and are not being used to apply a voltage drive signal $V_D$ to the subject S. Isolating the signal generator 117 from the drive electrode 113 removes the unintended return current path(s) that would otherwise be present due to the low output impedance of the amplifier $A_1$, thereby constraining current to flow only between the two selected drive electrodes 113. Other techniques may be used to achieve a similar effect, such as using an amplifier incorporating a high impedance output-disable state.

The current signal $I_S$ being applied to the subject S is detected and amplified using the amplifier $A_2$, with the amplified current signal $I_S$ being returned to the processing system 102, along the cable 953 and via the ADC 728.

Similarly, the sensor 118 operates by having the amplifier $A_3$ amplify the voltage detected at the second electrode 115, returning the amplified analogue sensed voltage signal $V_S$ along the cable 954, to the ADC 727.

The cables 951, 952, 953, 954, 955, 956 may be provided in a number of different configurations depending on the preferred implementation. In one example, each of the cables 951, 952, 953, 954, 955, 956 are provided in a single lead L, although this is not essential, and the cables could be provided in multiple leads, as will be described in more detail below.

Another potential source of error is caused by cross electrode capacitive coupling. As shown in Figure 9B, the relative proximity of the electrodes 113, 115 and the corresponding connections 961, 962, results in an effective capacitance $C_{DS}$, between the output of the drive amplifier $A_1$ and the input of the sense amplifier $A_3$. Accordingly, this will cause a parasitic current flow between the amplifiers electrodes $A_1$, $A_3$, which can in turn result in inaccuracies in the measurements, particularly at higher frequencies.

To cancel the cross electrode capacitive coupling a cross electrode capacitance cancelling circuit is provided, as shown in Figure 9C, which shows an equivalent circuit modelling the electrical responsiveness of the electrodes 113, 115 in use.

In this example, the impedances of each electrode 113, 115 and the subject S are represented by respective impedances $Z_{113}$, $Z_{115}$, $Z_S$, formed by respective resistor and capacitor arrangements. The cross electrode capacitance cancelling circuit 970 is coupled to the output of the drive amplifier $A_1$ and the input of the sense amplifier $A_3$, and includes an inverting amplifier $A_4$, having an input coupled to the output of the drive amplifier $A_1$. The output of the inverting amplifier is connected in series via a resistor $R_{10}$ and a capacitor $C_{10}$, to the input of the sense amplifier $A_3$.

In this arrangement any signal output from the drive amplifier $A_1$ will be inverted and then applied to the input of the sense amplifier $A_3$. By selecting appropriate values for the resistor $R_{10}$ and a capacitor $C_{10}$, this allows the inverted signal to have a magnitude equal to the magnitude of any signal resulting from the effective cross electrode capacitance $C_{DS}$.

In one example, the resistance and/or capacitance of the resistor $R_{10}$ and capacitor $C_{10}$ respectively, can be adjusted, through the use of suitable adjustable components, such as a variable resistor or capacitor. This allows the magnitude and/or phase of the inverted signal to be controlled so that it effectively cancels the signal resulting from the effective cross electrode capacitance $C_{DS}$. It will be appreciated that adjustment of the components may be performed during a calibration process, which will typically include the complete electrode unit together with its associated electrodes attached so that all parasitic capacitances are accurately represented.

Accordingly, the cross electrode capacitance cancelling circuit 970 provides an effective negative capacitance between the drive electrode 113 and corresponding sense electrode 115, so that a negative current flow occurs, thereby cancelling the parasitic current. This therefore negates the effect of any capacitive coupling between the drive and sense electrodes 113, 115. The electrode system may also include an input capacitance cancelling circuit, an example of which is shown in Figure 9D.

In use, the sense electrodes 115 can capacitively couple to the environment, which results in an effective input capacitance $C_{EI}$ at the input of the sense amplifier $A_3$. The effective capacitance allows signal leakage from the input of the sense amplifier to ground, thereby reducing the signal available at the amplifier input.

Accordingly, in this example, an input capacitance cancelling circuit 980 is provided which connects the positive amplifier input of the sense amplifier $A_3$ to the output of the sense amplifier, via a resistor $R_{11}$ and a capacitor $C_{11}$. This acts as a positive feedback loop, allowing a proportion of the amplified signal to be returned to the amplifier input. This acts to cancel the reduction in signal at the amplifier input that is caused by the effective input capacitance $C_{EI}$, and therefore provides an effective negative capacitance that cancels the effect of the effective input capacitance $C_{EI}$ at the amplifier input. Again, the input capacitance cancelling circuit requires tuning, which can be achieved during calibration by suitable adjustment of the values of the resistor $R_{11}$ and/or the capacitor $C_{11}$.

As briefly mentioned above, when separate leads 123, 125, are used for the voltage signal $V_S$ and the current signal $I_S$, then inductive coupling between the leads 123, 125 can result in EMFs being induced within the leads 123, 125. The magnitude of the EMF is dependent on the degree of coupling between the leads 123, 125 and hence their physical separation, and also increases in proportion to the frequency and amplitude of the current signal $I_S$.

The EMF induced within the leads 123, 125 results in an effective EMF across the input of the sensor 118. As a result, a component of the sensed voltage signal $V_S$ is due to the induced EMF, which in turn leads to inaccuracies in the determined voltage signal $V_S$ and the current signal $I_S$.

The effect of inductive coupling varies depending on the physical separation of the leads 123, 125. Accordingly, in one example, the effect of inductive coupling between leads can be reduced by physically separating the leads as much as possible. Thus, in one example, the cables 951, 952, 953, 954, 955, 956 are provided in separate physically separated leads. However, a problem with this arrangement is that the amount of inductive coupling will vary depending on the physical lead geometry, which can therefore vary between measurements. As a result, the magnitude of any inductive coupling can vary, making this difficult to account for when analysing the impedance measurements.

An alternative to using physically separate leads for each of the cables 951, 952, 953, 954, 955, 956 is to use a single combined lead L. The lead is formed so that the cables 951, 952, 953, 954, 955, 956 are held in a substantially constant relative physical configuration. In one example, the leads L are formed so as to provide a constant geometric arrangement by twisting each of the respective cables together. However, alternative fabrication techniques could be used such as making the leads from separate un-insulated shielded cables that are over moulded to maintain close contact.

As a result of the constant physical geometry, any EMF induced along the leads 123, 125 is substantially constant, allowing this to be accounted for during a calibration process.

Accordingly, when the measuring device 100 is initially configured, and in particular, when the algorithms are generated for analysing the voltage and current signals $V_S$, $Is$, to determine impedance measurements, these can include calibration factors that take into account the induced EMF. In particular, during the configuration process, a measuring device 100 can be used to take measurements from reference impedances, with the resulting calculations being used to determine the effect of the induced EMF, allowing this to be subtracted from future measurements.

A further issue with the lead arrangement is that of capacitive coupling between the respective cables, as will now be described with respect to Figure 10. For the purpose of this example, only cables 951, 953, 954 are shown for clarity.

In this example, the measuring device 100 is connected to the PCB's 950A, 950B to provide connections for each of the electrodes 113A, 113B, 115A, 115B. As also shown, each of the cables 951, 953, 954 have respective shielding 1051, 1053, 1054 provided thereon. The shielding is used to help prevent coupling between the respective cables 951, 953, 954. It will therefore be appreciated that the cables 951, 953, 954 are generally formed from a shielded wire core. In practice, the shielded cables may be 50Ω transmission lines, which minimize signal transmission distortion at high frequencies, thereby minimizing errors. In addition to this, the shields 1051, 1053, 1054 are typically interconnected at each end, to a reference voltage such as a ground, via respective connections 1055, 1056.

The use of shielded and grounded cables in this fashion helps reduce the effect of capacitive coupling, helping to further reduce inaccuracies in obtained measurements.

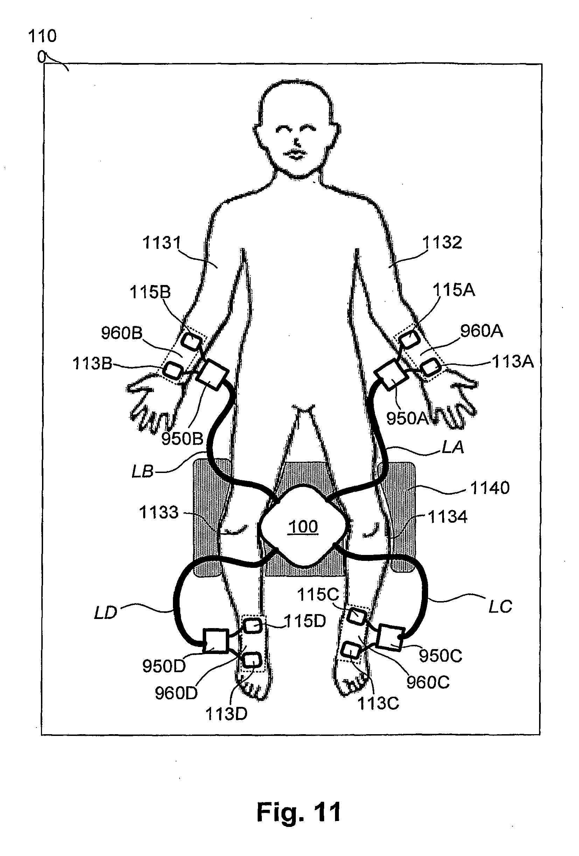

A further potential issue is that of inductive coupling between the different leads L, as well as capacitive coupling between the subject and the subject and the bed. In this regard, parasitic capacitances allow high frequency currents to bypass the intended current path through the body, resulting in measurement errors. To take this into account, in one example, the leads L for each electrode system can be physically separated as much as possible and/or provided in an arrangement that minimizes lead length in use. An example of an arrangement for achieving this will now be described with respect to Figure 11.

For the purpose of this example, the measuring system provides four measuring channels, designated by the suffixes A, B, C, D. It will be appreciated that this can be achieved by using a modified version of the measuring device 100, in which further ADCs 727, 728 and DACs 729 are provided as briefly described above.

In this example, the subject S is laying on a bed 1100, with arms 1131, 1132 positioned by the subject's side, and the legs 1133, 1134 resting on a support 1140, which incorporates the measuring device 100. The support may be any form of support, but is typically formed from moulded foam, or the like, which arranges the subject with the measuring device 100 positioned substantially between the subject's knees. The measuring device 100 is typically incorporated into the support both to ensure accurate location of the subject relative to the measuring device 100, and also to protect the subject S from damage caused by rubbing or other impact with a housing of the measuring device 100.

By providing a four channel arrangement, this allows a respective electrode system to be mounted to each of the subject's limbs. Thus, as shown, each limb 1131, 1132, 1133, 1134 has a respective substrate 760 mounted thereon, to thereby provide a drive and sense electrode 113, 115 on each wrist and ankle. The electrodes 113, 115, are coupled to respective signal generators and sensors mounted on the substrates 750, which are in turn coupled to the measuring device 100 via respective leads *LA, LB, LC, LD.*

The leads are arranged so that each lead *LA, LB, LC, LD* extends away from the measuring device 100 in different directions, thereby maximizing the physical separation of the leads and hence helping to reduce any inductive coupling therebetween.

Additionally, the leads *LA, LB, LC, LD* are preferably adapted to extend perpendicularly from both the measuring device 100 and the subject *S,* to thereby further reduce the effects of capacitive coupling.

Furthermore, by having the measuring device 100 positioned near the subject's knee, this places the measuring device 100 approximately equi-distant between the subject's wrists and ankles. Thus, by arranging the measuring device 100 towards the lower end of the bed 1100, this reduces the length of leads *LA, LB, LC, LD* needed to place the electrodes on the wrist and ankle of the subject S, whilst maintaining substantially equal lead lengths, which helps further reduce both inductive and capacitive coupling effects. In this regard, the EMF originating from any inductive coupling effect is proportional to the relevant lead length, thereby equalising any effect for the different leads. Similarly, capacitive coupling between the leads (ground) and the subject S, which can create current shunt paths, is also minimized.

The above described arrangement is for the purpose of example only, and it will be appreciated that in practice, any suitable mechanisms for positioning the measuring device 100 in the vicinity of the subject's upper legs (approximately midway between the wrists and ankles) can be used. Thus, for example, this could involve simply resting the measuring device 100 on the subject's legs, providing a custom built support, or the like.

It will be appreciated that in this arrangement, by having four first electrodes and four second electrodes positioned on the limbs, this allows a range of different limb and/or whole body impedance measurements to be performed.

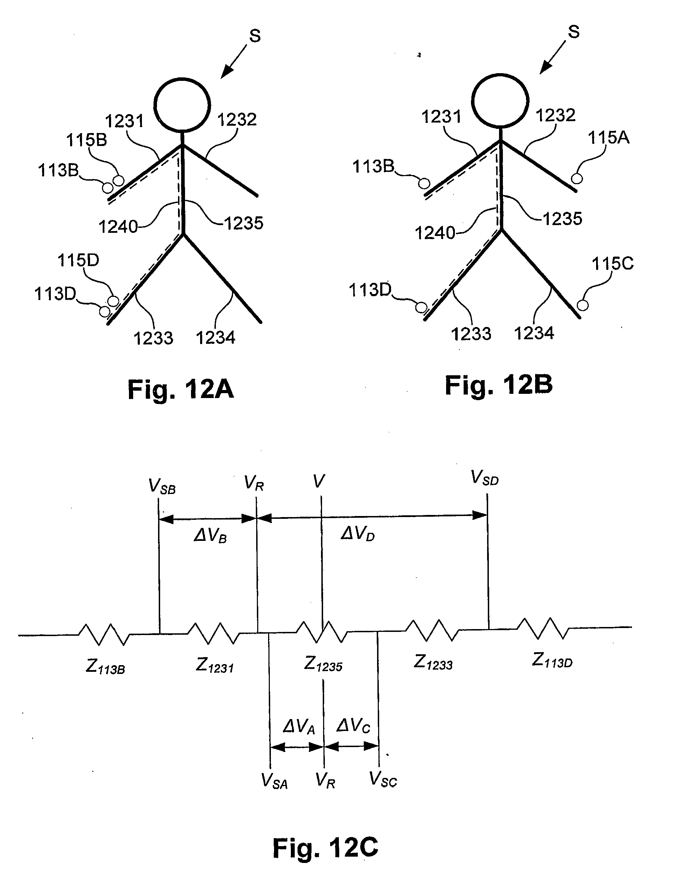

The electrode configuration shown in Figure 11 can be used to perform an alternative balancing process, as will now be described with reference to Figures 12A and 12B.

For the purpose of these examples, the subject *S* has arms 1231 1232, legs 1233, 1234 and a torso 1235 and the measuring device 300 (not shown for clarity) is provided in a multi-channel configuration similar to that shown in Figure 9, with respective pairs of drive and sense electrodes 113A, 115A; 113B, 115B; 113C, 115C; 113D, 115D provided on the wrist and ankles of the subject. In Figures 12A and 12B, active electrodes only are shown.

In each example, a drive electrode configuration is used that applies a drive signal to the drive electrodes 113B, 113D, so that the signal passes through the arm 1231, the torso 1235 and the leg 1233, as shown by the dotted line 1240.

In the example of Figure 12A sense electrodes 115B, 115D provided on the arm 1231 and on the leg 1233 are used to perform the balancing. In contrast, in the arrangement of Figure 12B, the sense electrodes 115A, 115C provided on the contra-lateral limbs 1232, 1234 are used to perform balancing. This leads to different effective electrical models for the balancing process, as shown in Figure 12C. The effective electrical model represents impedances encountered by the drive signal, including impedances $Z_{113B}$, $Z_{113D}$, $Z_{1231}$, $Z_{1235}$, $Z_{1233}$, representing the impedances of the drive electrode impedances 113B, 113D, the arm 1231, the torso 1235 and the leg 1233, respectively.

In the electrode configuration of Figure 12A, the sense electrodes are provided on the arm 1231 and the leg 1233, so that voltages induced within the subject are effectively sensed at the points between the drive electrodes 113B, 113D and the respective limb 1231, 1233. The sensed voltages measured at the electrodes 115B, 115D are shown at $V_{SB}$ and $V_{SD}$, respectively, and these effectively take into account current flow through the arm 1231, the torso 1235 and the leg 1233.

When performing balancing, the drive signal is controlled to minimise the common mode voltage such that $V_{SB} \approx - V_{SD}$. In this configuration, the effective ground reference voltage $V_R$ is electrically centred between the sensed voltages $V_{SB}$, $V_{SD}$, such that the differences $\Delta V_B$, $\Delta V_D$ between the reference voltage $V_R$ and each sensed voltage $V_{SB}$, $V_{SD}$ is approximately equal $\Delta V_B \approx \Delta V_D$. This therefore takes into account differences in impedances for the drive electrodes 113B, 113D, which typically arise from different contact impedances, so that if one of the electrodes has a significantly higher impedance than the other electrode, the signal applied to the body after the electrodes is still symmetrical with respect to the sense electrodes 115B, 115D.

As the impedance of the arm $Z_{1231}$ is generally higher than the torso impedance $Z_{1235}$ and leg impedance $Z_{1233}$, then generally the signal voltage difference across the arm 1231 is approximately equal to that across the torso 1235 and leg 1233 combined. Consequently, the location of the reference voltage $V_R$ does not generally occur at the geometric centre of the subject's body, but rather occurs somewhere near the shoulder region of the subject *S*. As a result, the subject's body centre voltage *Vc* is not necessarily minimised by balancing according to the sensed voltages $V_{SB}$, $V_{SD}$ and there can be a significant residual signal voltage *V* at the centre of the subject's torso 1235, which corresponds to the subject's body centre. Thus, the body centre voltage $V_C = V \neq V_R$. The residual signal voltage will result in current flow due to capacitive coupling between the subject and the environment, such as the bed on which the subject is positioned. This in turn impacts on the accuracy of the impedance measurements.

By contrast, the arrangement shown in Figure 12B senses the voltages in the subject using the sense electrodes 115A, 115C provided on the contralateral limbs 1232, 1234. As there is no current flow through the contralateral limbs 1232, 1234, the contralateral limbs 1232, 1234 are effectively at the same voltage along their entire length (i.e. isopotential). Accordingly, the sense electrodes 115A, 115C effectively measures the voltages at the point where the torso 1235 joins the arm 1231 and the leg 1233 as also shown in Figure 12C.

In this instance if the balancing is performed, the reference voltage $V_R$ is electrically centred between the sensed voltages $V_{SA}$, $V_{SC}$, such that the difference $\Delta V_A$, $\Delta V_C$ between the reference voltage $V_R$ and each sensed voltages $V_{SA}$, $V_{SC}$ is approximately equal $\Delta V_A \approx \Delta V_C$. As the voltage induced by the overall drive signal $V_D$ is measured across the torso only, and as the upper and lower torso have similar impedances, the reference voltage $V_R$ is positioned midway along the torso 1235. As the reference voltage is typically set to 0*V* this minimises the amplitude of the signal voltage on the torso 1235, as induced by the drive signal, which in turn reduces the effect of capacitive coupling between the subject and the bed.

Accordingly, whilst it will be appreciated that balancing can be performed using the configuration of Figure 12A, this typically only takes into account variations in electrode impedances of the drive electrodes 113B, 113D. Whilst this will also generally reduce the overall potential of the subject's torso, and hence reduce the effect of parasitic capacitances, it still does not necessarily result in the voltages in the body being balanced symmetrically with respect to the torso. Accordingly, in one example it is preferred to use the electrode configuration shown in Figure 12B.

Thus, balancing can be performed for a range of different electrode configurations, including sensing voltages on the same limbs to which the voltage drive signals are applied. However, in one example, the balancing is performed by passing signals along a first limb, the torso and a second limb with the voltage signals being measured by different third and fourth limbs. By measuring the voltages on different limbs, this ensures that balancing is performed about the subject's torso which in turn results in reduced effect of capacitive coupling between the subject and the environment.

It will be appreciated that in practice, there will always be some parasitic current flow from the torso even when the centre-body voltage is balanced. This is due to the relatively large physical size of the torso. However, the process of balancing the centre-body voltage attempts to minimise this error and also enables a repeatable reference point to be achieved.

Persons skilled in the art will appreciate that numerous variations and modifications will become apparent. All such variations and modifications which become apparent to persons skilled in the art, should be considered to fall within the spirit and scope that the invention broadly appearing before described.

For example, the above described technique can be used to determine values for the impedance parameters $R_0$ and $R_\infty$, and accordingly may be used in any scenario for which this information is useful. This can include for example, diagnosing the presence, absence or degree of a range of conditions and illnesses, including, but not limited to oedema, lymphoedema, or the like. The technique can also be used for performing body composition analysis, for example to examine relative fat levels or the like.

Whilst the above described examples use resistors, any suitable electronic components that can be used in simulating a subject's impedance response may be used. The term impedance is intended to cover any form of impedance measurement including resistance, reactance or admittance measurements.

**Claims**

1. Apparatus for use in performing impedance measurements on a subject, wherein the apparatus includes a processing system (102) for:

   a) determining impedance measurements of at least one body segment of the subject at more than three frequencies;
   b) determining combinations of the impedance measurements, each combination including impedance measurements at three frequencies;
   c) for each combination;

      i. at each of the three frequencies, determining (220, 540) first and second parameter values for first and second impedance parameters relating to the impedance measurements;
      ii. solving (230) simultaneous equations representing a circle defined with respect to the first and second impedance parameters to thereby determine circle parameter values, the equations being solved using the first and second parameter values at each of the three frequencies; and
      iii. using the circle parameter values to determine (240) a third impedance parameter value at a respective frequency; and,

   d) determining an indicator indicative of relative fluid levels within the body segment of the subject, the indicator being an average of the third impedance parameter values determined for each combination.

2. Apparatus according to claim 1, wherein the indicator is indicative of extra-cellular fluid levels and/or wherein the third impedance parameter is indicative of the impedance at zero frequency.

3. Apparatus according to claim 1 or claim 2, wherein the processing system (102) is for:

   a) determining third impedance parameter values at a respective frequency for each of first and second body segments;
   b) determining (550) a ratio using the third impedance parameter values; and,
   c) using the ratio to determine the indicator;

preferably wherein the first and second body segments are portions of contra-lateral limbs.

4.   Apparatus according to claim 3, wherein the processing system (102) is for:

a) comparing (570) the ratio to a reference; and,
b) using results of the comparison to determine the indicator, wherein the reference includes at least one of:

a predetermined threshold;
a tolerance determined from a normal population;
a predetermined range; and
an indicator previously determined for the subject.

5.   Apparatus according to any one of the claims 1 to 4, wherein the processing system (102) is for:

a) using the circle parameter values to determine a fourth impedance parameter value at a respective frequency;
b) using the third and fourth impedance parameters values to determine an index indicative of a ratio of the extra-cellular to intra-cellular fluid; and,
c) determining the indicator using the index.

6.   Apparatus according to claim 5, wherein the processing system (102) is for:

a) determining an index for first and second body segments; and,
b) determining an index ratio based on the index for the first and second body segments;

preferably wherein the first and second body segments are at least one of: different types of body segment, limbs, and a leg and an arm.

7.   Apparatus according to claim 5 and claim 6, wherein the processing system is for:

a) determining values for parameters $R_0$ and $R_\infty$ from the impedance parameter values; and,
b) calculating an index (I) using the equation:

$$I = \frac{R_\infty}{R_0 - R_\infty}$$

where:

$R_0$ is the resistance at zero frequency; and,
$R_\infty$ is the resistance at infinite frequency.

8.   Apparatus according to any one of the claims 1 to 7, wherein the processing system (102) is for, displaying an indication of at least one of:

a) the third impedance parameter values;
b) the first and second impedance parameter values;
c) the circle parameters;
d) a ratio of extra-cellular to intra-cellular fluid; and,
e) an indication of the at least one of the presence, absence or degree of tissue oedema in the subject.

9.   Apparatus according to any one of the claims 1 to 8, wherein the apparatus includes:

a) a signal generator (117A, 117B) for generating an alternating signal at each of a plurality of frequencies;
b) at least two supply electrodes (113A, 113B) for applying the generated alternating signal to a subject;
c) at least two measurement electrodes (115A, 115B) for detecting a signal across the subject; and,
d) a sensor (118A, 118B) coupled to the measurement electrodes for determining the signal across the subject, the sensor being coupled to the processing system to thereby allow the processing system to determine the measured impedances.

10. Apparatus according to any one of the claims 1 to 9, wherein the apparatus includes a number of electrode systems, and wherein each electrode system includes:

    a) a sensor (118A, 118B);
    b) a signal generator (117A, 117B);
    c) a first substrate (950) having the signal generator and sensor mounted thereon; and
    d) a second substrate (960) having at least two conductive pads (963, 965) mounted thereon, the conductive pads forming a first and a second electrode for coupling the signal generator and the sensor to a subject in use.

11. Apparatus according to claim 10, wherein the electrode system includes a capacitive cancelling circuit (970) for cancelling capacitive coupling between the first and second electrodes;
preferably wherein the capacitive cancelling circuit includes an inverting amplifier for coupling a signal generator output to a sensor input and the inverting amplifier applies a capacitive cancelling signal to the sensor input to thereby cancel any effective capacitance between the first electrode and the second electrode;
preferably wherein an inverting amplifier output is coupled to the sensor input via at least one of: a resistor (R10), a capacitor (C10), and an inductor, wherein at least one of the resistor and capacitor are adjustable, thereby allowing a capacitive cancelling signal applied to the sensor input to be controlled.

12. Apparatus according to claim 10 or claim 11, wherein the electrode system includes an input capacitance cancelling circuit for cancelling an effective input capacitance at a sensor input.

13. Apparatus according to any one of the claims 10 to 12, wherein the electrode system includes a feedback loop for connecting a sensor output to the sensor input, wherein the feedback loop includes at least one of: a resistor, a capacitor and an inductor, wherein at least one of the resistor and capacitor are adjustable, thereby allowing a current flow from the sensor output to the sensor input to be controlled;
preferably wherein the feedback loop applies an input capacitance cancelling signal to the sensor input to thereby cancel any effective capacitance at the sensor input.

14. Apparatus according to any one of the claims 1 to 13, wherein the processing system (102) includes a memory for storing software, and a processor operating under control of the software stored in the memory, and wherein the processor:

    a) determines impedance measurements of at least one body segment of the subject at more than three frequencies;
    b) determines combinations of the impedance measurements, each combination including impedance measurements at three frequencies;
    c) for each combination:

        i. determines (220, 540), at each of the three frequencies, first and second parameter values for first and second impedance parameters relating to the impedance measurements;
        ii. solves (230) simultaneous equations representing a circle defined with respect to the first and second impedance parameters to thereby determine circle parameter values, the equations being solved using the first and second parameter values at each of the three frequencies; and
        iii. uses the circle parameter values to determine (240) a third impedance parameter value at a respective frequency; and,

    d) determines an indicator indicative of relative fluid levels within the body segment of the subject, the indicator being an average of the third impedance parameter values determined for each combination.

15. A method for use in performing impedance measurements on a subject, wherein the method includes, in a processing system (102):

    a) determining impedance measurements of at least one body segment of the subject at more than three frequencies;
    b) determining combinations of the impedance measurements, each combination including impedance measurements at three frequencies;

c) for each combination:

i. at each of the three frequencies, determining (220, 540) first and second parameter values for first and second impedance parameters relating to the impedance measurements;
ii. solving (230) simultaneous equations representing a circle defined with respect to the first and second impedance parameters to thereby determine circle parameter values, the equations being solved using the first and second parameter values at each of the three frequencies; and
iii. using the circle parameter values to determine (240) a third impedance parameter value at a respective frequency; and,

d) determining an indicator indicative of relative fluid levels within the body segment of the subject, the indicator being an average of the third impedance parameter values determined for each combination.

**Patentansprüche**

1. Vorrichtung zur Verwendung bei der Durchführung von Impedanzmessungen an einem Patienten, wobei die Vorrichtung ein Verarbeitungssystem (102) für Folgendes umfasst:

a) Bestimmen von Impedanzmessungen von mindestens einem Körpersegment des Patienten bei mehr als drei Frequenzen;
b) Bestimmen von Kombinationen der Impedanzmessungen, wobei jede Kombination Impedanzmessungen bei drei Frequenzen umfasst;
c) für jede Kombination;

i. bei jeder der drei Frequenzen, Bestimmen (220, 540) von ersten und zweiten Parameterwerten für erste und zweite Impedanzparameter, die die Impedanzmessungen betreffen;
ii. Lösen (230) von simultanen Gleichungen, die einen Kreis darstellen, der in Bezug auf die ersten und zweiten Impedanzparameter definiert ist, um dadurch Kreisparameterwerte zu bestimmen, wobei die Gleichungen unter Verwendung der ersten und zweiten Parameterwerte bei jeder der drei Frequenzen gelöst werden; und
iii. Verwenden der Kreisparameterwerte, um einen dritten Impedanzparameterwert bei einer entsprechenden Frequenz zu bestimmen (240); und

d) Bestimmen eines Indikators, der relative Fluidpegel innerhalb des Körpersegments des Patienten anzeigt, wobei der Indikator einen Durchschnittswert der dritten Impedanzparameterwerte darstellt, die für jede Kombination bestimmt werden.

2. Vorrichtung nach Anspruch 1, wobei der Indikator extrazelluläre Fluidpegel anzeigt und/oder wobei der dritte Impedanzparameter die Impedanz bei der Frequenz null anzeigt.

3. Vorrichtung nach Anspruch 1 oder Anspruch 2, wobei das Verarbeitungssystem (102) für Folgendes dient:

a) Bestimmen von dritten Impedanzparameterwerten bei einer jeweiligen Frequenz für erste und zweite Körpersegmente;
b) Bestimmen (550) eines Verhältnisses unter Verwendung der dritten Impedanzparameterwerte; und
c) Verwenden des Verhältnisses, um den Indikator zu bestimmen; wobei das erste und zweite Körpersegment vorzugsweise Abschnitte von kontralateralen Gliedern sind.

4. Vorrichtung nach Anspruch 3, wobei das Verarbeitungssystem (102) für Folgendes dient:

a) Vergleichen (570) des Verhältnisses mit einer Referenz; und
b) Verwenden der Ergebnisse des Vergleichs zur Bestimmung des Indikators, wobei die Referenz mindestens eines von Folgendem umfasst:

einen vorbestimmten Schwellenwert;
eine Toleranz, die basierend auf einer normalen Population bestimmt wird;
einen vorbestimmten Bereich; und

einen Indikator, der zuvor für den Patienten bestimmt wurde.

**5.** Vorrichtung nach einem der Ansprüche 1 bis 4, wobei das Verarbeitungssystem (102) für Folgendes dient:

a) Verwenden der Kreisparameterwerte, um einen vierten Impedanzparameterwert bei einer entsprechenden Frequenz zu bestimmen;
b) Verwenden der dritten und vierten Impedanzparameterwerte, um einen Index zu bestimmen, der ein Verhältnis zwischen dem extrazellulären und dem innerzellulären Fluid anzeigt; und
c) Bestimmen des Indikators unter Verwendung des Index.

**6.** Vorrichtung nach Anspruch 5, wobei das Verarbeitungssystem (102) für Folgendes dient:

a) Bestimmen eines Index für das erste und zweite Körpersegment; und
b) Bestimmen eines Indexverhältnisses basierend auf dem Index des ersten und zweiten Körpersegments;

wobei das erste und zweite Körpersegment vorzugsweise mindestens eines von Folgendem umfasst: verschiedene Arten von Körpersegmenten, Gliedmaßen und ein Bein und ein Arm.

**7.** Vorrichtung nach Anspruch 5 und Anspruch 6, wobei das Verarbeitungssystem für Folgendes dient:

a) Bestimmen von Werten für die Parameter $R_0$ und $R_\infty$ ausgehend von den Impedanzparameterwerten; und
b) Berechnen eines Index (I) unter Verwendung der Gleichung:

$$I = \frac{R_\infty}{R_0 - R_\infty}$$

wobei:

$R_0$ der Widerstand bei der Frequenz null ist; und
$R_\infty$ der Widerstand bei unendlicher Frequenz ist.

**8.** Vorrichtung nach einem der Ansprüche 1 bis 7, wobei das Verarbeitungssystem (102) zum Anzeigen einer Angabe von mindestens einem von Folgendem dient:

a) den dritten Impedanzparameterwerten;
b) den ersten und zweiten Impedanzparameterwerten;
c) den Kreisparametern;
d) einem Verhältnis zwischen extrazellulärem und intrazellulärem Fluid; und
e) einer Anzeige der Anwesenheit, der Abwesenheit und/oder des Grades eines Gewebeödems in dem Patienten.

**9.** Vorrichtung nach einem der Ansprüche 1 bis 8, wobei die Vorrichtung Folgendes umfasst:

a) einen Signalgenerator (117A, 117B) zur Erzeugung eines Wechselsignals bei jeder einer Vielzahl von Frequenzen;
b) mindestens zwei Versorgungselektroden (113a, 113b) zum Anlegen des erzeugten Wechselsignals an einen Patienten;
c) mindestens zwei Messelektroden (115a, 115b) zum Erfassen eines Signals an dem Patienten; und
d) einen Sensor (118A, 118B), der mit den Messelektroden gekoppelt ist, um das Signal an dem Patienten zu bestimmen, wobei der Sensor mit dem Verarbeitungssystem gekoppelt ist, um dadurch dem Verarbeitungssystem zu ermöglichen, die gemessenen Impedanzen zu bestimmen.

**10.** Vorrichtung nach einem der Ansprüche 1 bis 9, wobei die Vorrichtung eine Anzahl von Elektrodensystemen umfasst und wobei jedes Elektrodensystem Folgendes umfasst:

a) einen Sensor (118A, 118B);
b) einen Signalgenerator (117A, 117B);

c) ein erstes Substrat (950), auf dem der Signalgenerator und der Sensor angebracht sind; und

d) ein zweites Substrat (960), auf dem mindestens zwei leitende Kontaktflächen (963, 965) angebracht sind, wobei die leitenden Kontaktflächen eine erste und eine zweite Elektrode bilden, um den Signalgenerator und den Sensor bei Gebrauch mit einem Patienten zu koppeln.

11. Vorrichtung nach Anspruch 10, wobei das Elektrodensystem eine Kapazitätsaufhebungsschaltung (970) zum Aufheben einer Kapazitätskopplung zwischen der ersten und zweiten Elektrode umfasst;

wobei die Kapazitätsaufhebungsschaltung vorzugsweise einen Umkehrverstärker zum Koppeln eines Signalgeneratorausgangs mit einem Sensoreingang umfasst und der Umkehrverstärker ein Kapazitätsaufhebungssignal an den Sensoreingang anlegt, um dadurch eine etwaige effektive Kapazität zwischen der ersten Elektrode und der zweiten Elektrode aufzuheben;

wobei vorzugsweise ein Umkehrverstärkerausgang mit dem Sensoreingang über mindestens eines von Folgendem gekoppelt ist:

einem Widerstand (R10), einem Kondensator (C10) oder einem Induktor, wobei mindestens der Widerstand oder der Kondensator einstellbar ist, wodurch ein Kapazitätsaufhebungssignal, das an den Sensoreingang angelegt wird, geregelt werden kann.

12. Vorrichtung nach Anspruch 10 oder Anspruch 11, wobei das Elektrodensystem eine Eingangskapazitätsaufhebungsschaltung zum Aufheben einer effektiven Eingangskapazität am Sensoreingang umfasst.

13. Vorrichtung nach einem der Ansprüche 10 bis 12, wobei das Elektrodensystem eine Rückführungsschleife zum Verbinden eines Sensorausgangs mit dem Sensoreingang umfasst, wobei die Rückführungsschleife mindestens einen Widerstand, einen Kondensator oder einen Induktor umfasst, wobei mindestens der Widerstand oder der Kondensator einstellbar ist, wodurch ein Stromfluss von dem Sensorausgang zu dem Sensoreingang geregelt werden kann;

wobei vorzugsweise die Rückführungsschleife ein Eingangskapazitätsaufhebungssignal an den Sensoreingang anlegt, um dadurch etwaige effektive Kapazitäten am Sensoreingang aufzuheben.

14. Vorrichtung nach einem der Ansprüche 1 bis 13, wobei das Verarbeitungssystem (102) Folgendes umfasst: einen Speicher zum Speichern von Software und einen Prozessor, der unter der Steuerung der Software arbeitet, die in dem Speicher gespeichert ist, und wobei der Prozessor:

a) a) Impedanzmessungen von mindestens einem Körpersegment des Patienten bei mehr als drei Frequenzen bestimmt;

b) Kombinationen der Impedanzmessungen bestimmt, wobei jede Kombination Impedanzmessungen bei drei Frequenzen umfasst;

c) für jede Kombination:

i. bei jeder der drei Frequenzen erste und zweite Parameterwerte für die ersten und zweiten Impedanzparameter in Bezug auf die Impedanzmessungen bestimmt (220, 540);

ii. simultane Gleichungen löst (230), die einen Kreis darstellen, der in Bezug auf die ersten und zweiten Impedanzparameter definiert ist, um dadurch Kreisparameterwerte zu bestimmen, wobei die Gleichungen unter Verwendung der ersten und zweiten Parameterwerte bei jeder der drei Frequenzen gelöst werden; und

iii. die Kreisparameterwerte verwendet, um einen dritten Impedanzparameterwert bei einer entsprechenden Frequenz zu bestimmen (240); und

d) einen Indikator bestimmt, der relative Fluidpegel innerhalb des Körpersegments des Patienten anzeigt, wobei der Indikator einen Durchschnittswert der dritten Impedanzparameterwerte darstellt, die für jede Kombination bestimmt werden.

15. Verfahren zur Verwendung bei der Durchführung von Impedanzmessungen an einem Patienten, wobei das Verfahren in einem Verarbeitungssystem (102) Folgendes umfasst:

a) Bestimmen von Impedanzmessungen von mindestens einem Körpersegment des Patienten bei mehr als drei Frequenzen;

b) Bestimmen von Kombinationen der Impedanzmessungen, wobei jede Kombination Impedanzmessungen bei drei Frequenzen umfasst;

c) für jede Kombination:

i. bei jeder der drei Frequenzen, Bestimmen (220, 540) von ersten und zweiten Parameterwerten für die ersten und zweiten Impedanzparameter in Bezug auf die Impedanzmessungen;
ii. Lösen (230) von simultanen Gleichungen, die einen Kreis darstellen, der in Bezug auf die ersten und zweiten Impedanzparameter definiert ist, um dadurch Kreisparameterwerte zu bestimmen, wobei die Gleichungen unter Verwendung der ersten und zweiten Parameterwerte bei jeder der drei Frequenzen gelöst werden; und
iii. Verwenden der Kreisparameterwerte, um einen dritten Impedanzparameterwert bei einer entsprechenden Frequenz zu bestimmen (240); und

d) Bestimmen eines Indikators, der relative Fluidpegel innerhalb des Körpersegments des Patienten anzeigt, wobei der Indikator einen Durchschnittswert der dritten Impedanzparameterwerte darstellt, die für jede Kombination bestimmt werden.

## Revendications

1. Appareil destiné à être utilisé dans la réalisation de mesures d'impédance chez un sujet, l'appareil incluant un système de traitement (102) pour :

a) déterminer des mesures d'impédance d'au moins un segment corporel du sujet à plus de trois fréquences ;
b) déterminer des combinaisons pour les mesures d'impédance, chaque combinaison incluant des mesures d'impédance à trois fréquences ;
c) pour chaque combinaison, les opérations consistant à :

i. à chacune des trois fréquences, déterminer (220, 540) des première et deuxième valeurs paramétriques pour des premier et deuxièmes paramètres d'impédance relatifs aux mesures d'impédance ;
ii. résoudre (230) des équations simultanées représentant un cercle défini par rapport aux premier et deuxièmes paramètres d'impédance afin de déterminer par conséquent des valeurs paramétriques de cercle, les équations étant résolues grâce à l'utilisation des première et deuxième valeurs paramétriques à chacune des trois fréquences ; et
iii. utiliser les valeurs paramétriques de cercle pour déterminer (240) une troisième valeur paramétrique d'impédance à une fréquence respective ; et

d) déterminer un indicateur lequel indique des niveaux de fluide relatifs à l'intérieur du segment corporel du sujet, l'indicateur étant une moyenne des troisièmes valeurs paramétriques d'impédance ayant été déterminées pour chaque combinaison.

2. Appareil selon la revendication 1, l'indicateur étant indicatif de niveaux de fluide extra-cellulaire, et/ou le troisième paramètre d'impédance étant indicatif de l'impédance à la fréquence zéro.

3. Appareil selon la revendication 1 ou la revendication 2, le système de traitement (102) étant destiné à :

a) déterminer des troisièmes valeurs paramétriques d'impédance à une fréquence respective pour chacun des premier et deuxième segments corporels ;
b) déterminer (550) un rapport grâce à l'utilisation des troisièmes valeurs paramétriques d'impédance ; et
c) utiliser le rapport pour déterminer l'indicateur ;

de préférence, cas dans lequel les premier et deuxième segments corporels sont des portions de membres contra-latéraux.

4. Appareil selon la revendication 3, le système de traitement (102) étant destiné à :

a) comparer (570) le rapport à une référence ; et
b) utiliser les résultats de la comparaison pour déterminer l'indicateur, cas dans lequel la référence inclut au moins l'un des éléments suivants :

un seuil prédéterminé ;
une tolérance ayant été déterminée à partir d'une population normale ;
une gamme prédéterminée ; et
un indicateur qui a été déterminé antérieurement pour le sujet.

5. Appareil selon l'une quelconque des revendications 1 à 4, le système de traitement (102) étant destiné à :

a) utiliser les valeurs paramétriques de cercle pour déterminer une quatrième valeur paramétrique d'impédance à une fréquence respective ;
b) utiliser les troisième et quatrième valeurs paramétriques d'impédance pour déterminer un indice lequel indique un rapport entre le fluide extra-cellulaire et le fluide intra-cellulaire ; et
c) déterminer l'indicateur grâce à l'utilisation de l'indice.

6. Appareil selon la revendication 5, le système de traitement (102) étant destiné à :

a) déterminer un indice pour les premier et deuxième segments corporels ; et
b) déterminer un rapport d'indice sur la base de l'indice pour les premier et deuxième segments corporels ;

de préférence, cas dans lequel les premier et deuxième segments corporels sont au moins l'un des suivants, à savoir : différents types de segments corporels, de membres, et une jambe et un bras.

7. Appareil selon la revendication 5 et la revendication 6, le système de traitement étant destiné à :

a) déterminer des valeurs pour les paramètres $R_0$ et $R_\infty$ à partir des valeurs paramétriques d'impédance ; et
b) calculer un indice (I) grâce à l'utilisation de l'équation suivante :

$$I = \frac{R_\infty}{R_0 - R_\infty}$$

où :

$R_0$ représente la résistance à la fréquence zéro ; et
$R_\infty$ représente la résistance à la fréquence infinie.

8. Appareil selon l'une quelconque des revendications 1 à 7, le système de traitement (102) étant destiné à afficher une indication de l'un au moins des éléments suivants :

a) les troisièmes valeurs paramétriques d'impédance ;
b) les première et deuxième valeurs paramétriques d'impédance ;
c) les paramètres de cercle ;
d) un rapport entre le fluide extra-cellulaire et le fluide intra-cellulaire ; et
e) une indication de l'un au moins des éléments suivants, à savoir la présence, l'absence ou un certain degré d'oedème tissulaire chez le sujet.

9. Appareil selon l'une quelconque des revendications 1 à 8, l'appareil incluant :

a) un générateur de signaux (117A, 117B) pour générer un signal alternant à chaque fréquence d'une pluralité de fréquences ;
b) au moins deux électrodes d'alimentation (113A, 113B) pour appliquer à un sujet le signal alternant généré ;
c) au moins deux électrodes de mesure (115A, 115B) pour détecter un signal au niveau du sujet ; et
d) un capteur (118A, 118B) couplé aux électrodes de mesure pour déterminer le signal au niveau du sujet, le capteur étant couplé au système de traitement afin de permettre par conséquent au système de traitement de déterminer les impédances mesurées.

10. Appareil selon l'une quelconque des revendications 1 à 9, l'appareil incluant un certain nombre de systèmes à électrodes, et chaque système à électrodes incluant :

a) un capteur (118A, 118B) ;

b) un générateur de signaux (117A, 117B) ;

c) un premier substrat (950) sur lequel sont montés le générateur de signaux et le capteur ; et

d) un deuxième substrat (960) sur lequel sont montés au moins deux patins conducteurs (963, 965), les patins conducteurs formant une première et une deuxième électrodes pour coupler, à un sujet, le générateur de signaux et le capteur, lors de leur utilisation.

11. Appareil selon la revendication 10, le système à électrodes incluant un circuit d'annulation capacitif (970) pour annuler le couplage capacitif entre les première et deuxième électrodes ;

de préférence, cas dans lequel le circuit d'annulation capacitif inclut un amplificateur inverseur pour coupler une sortie du générateur de signaux à une entrée du capteur, et l'amplificateur inverseur applique un signal d'annulation capacitif à l'entrée du capteur afin d'annuler par conséquent toute capacité effective entre la première électrode et la deuxième électrode ;

de préférence, cas dans lequel une sortie d'amplificateur inverseur est couplée à l'entrée du capteur par l'intermédiaire de l'un au moins des postes suivants à savoir : une résistance (R10), un condensateur (C10) et un inducteur, cas dans lequel l'un au moins des postes suivants, à savoir la résistance et le condensateur, est réglable, ce qui permet par conséquent de piloter un signal d'annulation capacitif appliqué à l'entrée du capteur.

12. Appareil selon la revendication 10 ou la revendication 11, le système à électrodes incluant un circuit d'annulation de la capacité d'entrée afin d'annuler une capacité d'entrée effective au niveau d'une entrée de capteur.

13. Appareil selon l'une quelconque des revendications 10 à 12, le système à électrodes incluant une boucle de rétroaction pour connecter une sortie de capteur à l'entrée de capteur, cas dans lequel la boucle de rétroaction inclut l'un au moins des postes suivants à savoir : une résistance, un condensateur et un inducteur, cas dans lequel l'un au moins des postes suivants, à savoir la résistance et le condensateur, est réglable, ce qui permet par conséquent de piloter un flux de courant se rendant de la sortie de capteur à l'entrée de capteur ;

de préférence, cas dans lequel la boucle de rétroaction applique, à l'entrée de capteur, un signal d'annulation de la capacité d'entrée afin d'annuler par conséquent toute capacité d'entrée effective au niveau de l'entrée du capteur.

14. Appareil selon l'une quelconque des revendications 1 à 13, le système de traitement (102) incluant une mémoire pour stocker du logiciel, et un processeur qui fonctionne sous le pilotage du logiciel stocké dans la mémoire, et cas dans lequel le processeur :

a) détermine des mesures d'impédance d'au moins un segment corporel du sujet à plus de trois fréquences ;

b) détermine des combinaisons pour les mesures d'impédance, chaque combinaison incluant des mesures d'impédance à trois fréquences ;

c) pour chaque combinaison, les opérations consistant à :

i. déterminer (220, 540), à chacune des trois fréquences, des première et deuxième valeurs paramétriques pour des premier et deuxièmes paramètres d'impédance relatifs aux mesures d'impédance ;

ii. résoudre (230) des équations simultanées représentant un cercle défini par rapport aux premier et deuxièmes paramètres d'impédance afin de déterminer par conséquent des valeurs paramétriques de cercle, les équations étant résolues grâce à l'utilisation des première et deuxième valeurs paramétriques à chacune des trois fréquences ; et

iii. utiliser les valeurs paramétriques de cercle pour déterminer (240) une troisième valeur paramétrique d'impédance à une fréquence respective ; et

d) déterminer un indicateur lequel indique des niveaux de fluide relatifs à l'intérieur du segment corporel du sujet, l'indicateur étant une moyenne des troisièmes valeurs paramétriques d'impédance ayant été déterminées pour chaque combinaison.

15. Procédé destiné à être utilisé dans la réalisation de mesures d'impédance chez un sujet, le procédé incluant, dans un système de traitement (102), les opérations consistant à :

a) déterminer des mesures d'impédance d'au moins un segment corporel du sujet à plus de trois fréquences ;

b) déterminer des combinaisons pour les mesures d'impédance, chaque combinaison incluant des mesures d'impédance à trois fréquences ;

c) pour chaque combinaison, les opérations consistant à :

i. à chacune des trois fréquences, déterminer (220, 540) des première et deuxième valeurs paramétriques pour des premier et deuxièmes paramètres d'impédance relatifs aux mesures d'impédance ;

ii. résoudre (230) des équations simultanées représentant un cercle défini par rapport aux premier et deuxièmes paramètres d'impédance afin de déterminer par conséquent des valeurs paramétriques de cercle, les équations étant résolues grâce à l'utilisation des première et deuxième valeurs paramétriques à chacune des trois fréquences ; et

iii. utiliser les valeurs paramétriques de cercle pour déterminer (240) une troisième valeur paramétrique d'impédance à une fréquence respective ; et

d) déterminer un indicateur lequel indique des niveaux de fluide relatifs à l'intérieur du segment corporel du sujet, l'indicateur étant une moyenne des troisièmes valeurs paramétriques d'impédance ayant été déterminées pour chaque combinaison.

**Fig. 1**

Apply signals to
subject — 200

Determine signals
measured across
subject — 210

Determine first and
second impedance
*parameter* values at
three frequencies — 220

Solve simultaneous
equations to
determine circle
parameters — 230

Determine theoretical
impedance value from
circle parameters — 240

Determine indicator
indicative of fluid
levels — 250

# Fig. 2

Fig. 3

Fig. 4

Determine subject
details

500

Determine
affected limb

510

Position impedance
electrodes on the
subject S

520

Determine
impedance
measurements at
three frequencies

530

Derive impedance
parameters

540

Determine a limb
impedance ratio *IR*

550

Select reference

560

Compare limb
impedance ratio to
reference

570

Display
representation of
comparison

580

# Fig. 5

**Fig. 6A**

**Fig. 6B**

**Fig. 6C**

**Fig. 6D**

**Fig. 7**

```
┌─────────────────┐
│ Select impedance │        800
│measurement type │
└─────────────────┘
         │
         ▼
┌─────────────────┐
│  Load desired   │
│   software in   │        805
│ mesauring device│
│       100       │
└─────────────────┘
         │
         ▼
┌─────────────────┐
│   Select next   │
│   measurement   │        810
│  frequency fᵢ   │
└─────────────────┘
         │
         ▼
┌─────────────────┐
│Generate sequence of│
│digital control signals│   815
│ using processing │
│    system 102    │
└─────────────────┘
         │
         ▼
┌─────────────────┐
│ Convert control │
│signals using DAC│        820
│       724       │
└─────────────────┘
         │
         ▼
┌─────────────────┐
│Transfer analogue│
│control signals to│       825
│signal generator │
│       117       │
└─────────────────┘
         │
         ▼
┌─────────────────┐
│Generate voltage │
│signals Vₐₐ, Vₒ_B and│   830
│apply to subject │
└─────────────────┘
         │
         ▼
      ⬡ 835
```

910

895

# Fig. 8A

830

835 — Sense voltages $V_{SA}$, $V_{SB}$ induced across the subject using sensor 118

845 — Sense current signals $I_{SA}$, $I_{SB}$ using the signal generator 117

840 — Digitise the voltage signals $V_{SA}$, $V_{SB}$ using the ADC 722

850 — Digitise the current signals $I_{SA}$, $I_{SB}$ using the ADC 723

855 — Receive digitised current and voltage signals in the processing system 102

860 — Determine magnitude of applied current

865 — Compare applied current to threshold

870 — Is threshold exceeded?

Yes

875 — Terminate measuring process and generate alert

No

880

Fig. 8B

38

```
            ┌─────────┐
            │   870   │
            └────┬────┘
                 │
                 ▼
        ┌─────────────────┐
        │ Determine additive │      880
        │  voltage sensed    │
        └────────┬─────────┘
                 │
                 ▼
        ┌─────────────────┐
        │ Compare additive  │      885
        │voltage to threshold│
        └────────┬─────────┘
                 │
                 ▼
 ┌──────────────┐      Yes    ◇─────────◇
 │Generate modified│◄──────────│Is threshold│    890
 │control signals  │           │ exceeded? │
 │using processing │           ◇─────────◇
 │  system 102     │               │
 └───────┬────────┘              No│
   895   │                         ▼
         ▼                ┌─────────────────┐
     ┌────────┐          │   Determine      │    900
     │  820   │          │differential voltage│
     └────────┘          │    sensed         │
                         └────────┬─────────┘
                                  │
                                  ▼
                         ┌─────────────────┐
                         │Determine ratio and│   905
                         │ phase signals at  │
                         │applied frequency fi│
                         └────────┬─────────┘
                                  │
                                  ▼
              No          ◇─────────◇
          ┌──────────────│ Required  │          910
          │              │frequencies│
          ▼              │ complete  │
      ┌────────┐         ◇─────────◇
      │  810   │              │Yes
      └────────┘              ▼
                    ┌─────────────────┐
                    │ Transfer ratio and│        915
                    │ phase signals to the│
                    │ processing system   │
                    │for further processing│
                    └─────────────────┘
```

# Fig.8C

**Fig. 9A**

EP 2 348 986 B1

961    962

$A_1$

$A_3$

$C_{DS}$

113    115

S

**Fig. 9B**

970

$-A_4$    $R_{10}$    $C_{10}$

$A_1$    $A_3$

$C_{DS}$

$Z_{113}$    $Z_{115}$

$Z_S$    **Fig. 9C**

**Fig. 9D**

**Fig. 10**

**Fig. 11**

**Fig. 12A**

**Fig. 12B**

**Fig. 12C**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0079255 A
- WO 2005122888 A

- EP 1080686 A1